# FASCICULE DE BREVET EUROPEEN

(11) **EP 3 421 092 B1**
(45) Date de publication et mention de la délivrance du brevet: **22.04.2020**
(21) Numéro de dépôt: 18185253.4
(22) Date de dépôt: 07.05.2014
(51) Int. Cl.: A61P 1/04, A23L 29/212, A61K 36/48, A23L 29/231, A23L 29/238, A23L 33/00

(54) **COMPOSITION ANTI-REGURGITATION PRESERVANT LE TRANSIT INTESTINAL**
ANTI-REGURGITATIONSZUSAMMENSETZUNG, DIE DIE DARMTÄTIGKEIT AUFRECHTERHÄLT
ANTI-REGURGITATION COMPOSITION PRESERVING GUT MOTILITY

(30) Priorité: 07.05.2013 FR 1354200
(43) Date de publication de la demande: 02.01.2019
(62) Demande divisionnaire de: 14726894.0
(73) Titulaire: United Pharmaceuticals S.A., 75008 Paris (FR)
(72) Inventeur: MARGOSSIAN, Jonathan Albert, 1204 GENEVE (CH); PRADEAU, Nicolas, 35410 DOMLOUP (FR)
(74) Mandataire: Cabinet Becker et Associés

(56) Documents cités:
- EP-A1- 0 846 422
- EP-A1- 1 070 457
- WO-A1-2009/065900
- WO-A1-2010/120172
- WO-A1-2012/080462
- FR-A1- 2 913 857
- US-A1- 2004 258 825

## Description

### DOMAINE DE L'INVENTION

Le domaine de la présente invention est celui de la médecine et de la nutrition humaine, en particulier de la nutrition infantile. L'invention concerne des compositions nutritionnelles comme indiquées dans les revendications destinées à prévenir ou traiter les régurgitations et/ou le reflux gastro-œsophagien chez les nourrissons et enfants en bas âge sans altérer, voire en améliorant, leur transit intestinal, typiquement en réduisant ou évitant les effets secondaires (constipations, selles liquides, etc.) classiquement provoqués par l'ingestion de lait infantile à visée anti-régurgitation. Elle concerne également des compositions nutritionnelles destinées à prévenir et/ou traiter les troubles intestinaux du nourrisson ou de l'enfant.

L'invention a idéalement pour but de réduire, de préférence d'éliminer, les phénomènes de régurgitation et/ou de reflux affectant un sujet tout en préservant son transit intestinal, c'est-à-dire en ne provoquant chez lui ni diarrhée ni constipation. Des compositions, formules, préparations et laits infantiles anti-régurgitation et/ou anti-reflux préservant un transit intestinal correct chez les nourrissons et enfants en bas âge, en particulier évitant les anomalies du transit intestinal décrites dans le présent texte, sont ainsi plus particulièrement décrits.

### ART ANTERIEUR

Les régurgitations constituent un symptôme fréquemment observé chez les nouveau-nés et nourrissons résultant d'une augmentation de la pression abdominale par rapport à la pression thoracique. Elles surviennent souvent après les repas ou lors de phénomènes d'éructations. Ces régurgitations, qui peuvent également affecter l'adulte, et se distinguent, au sens médical du terme, des vomissements, sont sans conséquence sur le développement de l'enfant et relèvent surtout de l'inconfort.

Le mécanisme des régurgitations peut être expliqué de la manière suivante : le sphincter œsophagien inférieur est un muscle circulaire situé dans la partie inférieure de l'œsophage. Lors des repas, ce muscle se relâche de manière à faciliter la propulsion des aliments ingérés (bol alimentaire) dans l'estomac. La fonction principale de ce muscle est de prévenir le retour du contenu de l'estomac vers l'œsophage grâce à sa constriction tonique.

Chez le nourrisson, ce muscle peut être immature. Il ne peut donc pas toujours soutenir et compenser la pression exercée par le contenu gastrique. Il a alors tendance, pendant et après les repas, à se relâcher et à laisser remonter de petits volumes de liquide gastrique dans le pharynx et vers la bouche, via l'œsophage : les régurgitations.

Ce phénomène apparaît généralement dès les premières semaines de la vie de l'enfant. Les facteurs de développement physiologique qui contribuent à l'apparition de ces régurgitations disparaissent, le plus souvent spontanément, lorsque l'enfant atteint l'âge de 12 à 15 mois (Vandenplas Y., Belli D. et al, Current concepts and issues in the management of regurgitation of infants : a reappraisal, Acta Pediatr 85 : 531-534, 1996).

Les régurgitations sont souvent à l'origine de l'inquiétude des parents qui, de ce fait, n'hésitent pas à consulter un médecin. Les préoccupations varient, chez l'enfant, de la simple gêne, occasionnée par les renvois, jusqu'à la douleur (la paroi de l'œsophage étant irritée, cela donne lieu à des brûlures voire à des ulcérations de l'œsophage). Ces douleurs sont sources de pleurs et compliquent souvent l'endormissement de l'enfant.

Dans certains cas, un reflux excessivement fréquent et/ou prolongé peut être responsable de complications, définissant, quel que soit l'âge du sujet atteint, un Reflux Gastro-Oesophagien pathologique (RGO, encore identifié dans le présent texte sous le terme « reflux »). Chez le nourrisson et l'enfant en particulier, le reflux pathologique peut avoir des conséquences œsophagiennes et respiratoires plus atypiques : cassure de la courbe staturo-pondérale, rhino-pharyngo-bronchite récidivante, asthme et bronchiolite, anémie. On estime que les régurgitations et reflux surviennent chez environ 75% des enfants de moins de un an.

Le traitement recommandé par la Société Européenne de Pédiatrie, Gastro-entérologie, Hépatologie et Nutrition (ESPGHAN) consiste en un épaississement du bol alimentaire. Il a en effet été prouvé par des tests cliniques, qu'une augmentation de la viscosité du contenu gastrique peut réduire de manière significative la fréquence et le volume des régurgitations. Les thérapeutiques médicamenteuses (prokinétiques, antisécrétoires, etc.) sont essentiellement réservées au reflux pathologique.

Des épaississants classiquement employés depuis de très nombreuses années comme les amidons précuits et/ou prégélatinisés ou la farine de graine de caroube par exemple peuvent être ajoutés, lors de la préparation du biberon, au lait infantile.

Des laits infantiles pré-épaissis dits laits « anti-régurgitation » (AR) sont proposés depuis plusieurs années. Ces formulations AR contiennent déjà un agent épaississant, choisi parmi les amidons et la gomme de caroube, à l'origine de la viscosité du lait reconstitué et ayant ainsi un effet anti régurgitations.

WO2012/080462 décrit une composition anti-régurgitation et/ou anti-reflux, comprenant au moins une pectine faiblement estérifée, et au moins un épaississant et/ou gélifiant choisi par exemple parmi la gomme xanthane, la carboxy-méthyle cellulose, l'hydroxy-propyle cellulose, l'hydroxy-méthyle cellulose, une carraghénane, un alginate, la gomme de guar et la farine de graine de caroube. Cette composition peut avantageusement comprendre en outre une pectine fortement estérifiée.

Toutefois ces laits présentent des inconvénients : ils ont très souvent des effets secondaires sur le transit intestinal. De nombreux cas de constipation ou au contraire de selles trop liquides et/ou trop fréquentes chez l'enfant nourri au lait infantile AR sont rapportés.

On suppose que ces effets sont dus aux épaississants utilisés : les préparations à base de farine de caroube sont connues pour entraîner des douleurs abdominales, coliques et diarrhées suite à la fermentation de la caroube dans le colon (Carré et Al, Arch Dis Child, 1985, 60, 71-75). La caroube induit des selles fréquentes, liquides et gélatineuses. Bien que rares, de sérieuses complications telles que obstruction intestinale aigüe ou entérocolite nécrosante ont été rapportées chez des prématurés et des nouveaux nés (Vandenplas et AL The Diagnosis and management of gastro-oesophogeal reflux in infants. Early Hum dev 2005 ; 81 : 1011-24). Les selles liquides sont également sources d'inquiétudes pour les parents. Les diarrhées peuvent en effet entraîner des pertes accrues en eau et électrolytes (sodium, potassium, bicarbonates) ce qui peut vite devenir très problématique chez un enfant en bas âge, en entraînant une déshydratation plus ou moins sévère, et nécessiter une réhydratation.

Les laits infantiles épaissis avec de l'amidon sont plutôt connus pour entraîner des problèmes de constipation.

La constipation peut se définir par des difficultés, par un retard ou une absence totale de défécation, pendant deux semaines ou plus, suffisants pour provoquer une détresse significative chez le sujet qui en souffre (Baker et Al, J Pediatric Gastroenterol Nutr 1999 ; 29 :612-26). En effet, plus le retard à la défécation est long, plus l'expulsion des selles sera difficile et douloureuse, et parfois à l'origine de fissures anales chez le nourrisson ou l'enfant. Cette souffrance, source d'inconfort quand elle n'est pas source de douleurs chez les nourrissons et enfants, est souvent également source d'inquiétudes chez les parents qui redoutent qu'elle soit le symptôme d'un problème médical grave. La constipation peut également se caractériser par l'émission de selles dures, la dureté pouvant être aisément évaluée par l'homme du métier au regard de l'échelle de Bristol (types 1, 2 et 3 de ladite échelle).

Novalac AR® est un exemple de lait épaissi à l'amidon [Ingrédients : Lait écrémé, lactose, huiles végétales, amidon, minéraux, vitamines, L-cystine, taurine, bitartrate de choline, inositol, anti-oxydant (tocophérols), L-carnitine]. Ce lait donne de bons résultats en termes d'effet sur les régurgitations, mais entraîne des problèmes de constipation chez un certain nombre d'enfants.

On pourrait donc penser qu'une composition anti-régurgitation comprenant de l'amidon et de la caroube (connue pour induire des selles liquides, comme expliqué ci-dessus) prémunirait le nourrisson contre les effets secondaires respectivement induits sur les selles par les deux types d'épaississants, les effets de ceux-ci s'annihilant l'un l'autre. Il n'en est rien : une étude Novalac a également montré que près de 18% des enfants recevant une formule épaissie avec de la caroube soluble à froid et de l'amidon [AR Digest® - Ingrédients : Maltodextrines, huiles végétales, lactose, hydrolysat de protéines de lait, caroube, glucose, amidon, minéraux, vitamines, anti-oxydant (tocophérols), taurine, inositol, L-carnitine] présentaient des selles diarrhéiques (Enquête Qualinov. Nutrition et Pédiatrie. Janvier 2010, 4, 10).

Il existe donc un certain nombre de laits infantiles anti-régurgitation, mais ces laits présentent des effets secondaires indésirables sur le transit intestinal. Il n'existe pas à l'heure actuelle de lait infantile, typiquement de lait infantile anti-régurgitation et/ou anti-reflux, qui permette à l'enfant de préserver son transit intestinal ou, en d'autres termes, qui limite ou empêche l'altération (quelle qu'elle soit) de ce transit, encore moins qui soit capable de rétablir un transit normal lorsque ce dernier est altéré. La mise au point d'un tel lait s'avère donc un véritable besoin pour améliorer le confort de l'enfant comme celui de ses parents et permettre une meilleure observance au traitement diététique des régurgitations.

De manière avantageuse, les inventeurs ont mis au point une composition nutritionnelle, en particulier une composition nutritionnelle anti-régurgitation et/ou anti-reflux, qui préserve, idéalement restaure, de manière surprenante et inattendue, le transit intestinal et évite ainsi au nourrisson les désagréments associés à une altération dudit transit tels que constipation, selles molles, selles liquides et/ou trop fréquentes (diarrhée), et/ou coliques.

### RESUME DE L'INVENTION

L'objet de l'invention est une composition nutritionnelle, telle que définie dans les revendications, typiquement une composition anti-régurgitation et/ou anti-reflux (ci-dessous désignée plus généralement en tant que « composition nutritionnelle anti-régurgitation » ou « composition anti-régurgitation »), pour nourrisson ou enfant, laquelle de préférence altère moins que les compositions de l'art antérieur, idéalement n'altère pas, le transit intestinal dudit nourrisson ou dudit enfant, ou en d'autres termes permet de préserver son transit intestinal et donc de limiter ou réduire, idéalement d'éviter, la constipation, les selles molles, les selles liquides, les selles trop fréquentes et/ou les coliques, de préférence la constipation, les selles molles, les selles liquides et/ou trop fréquentes. La composition comprend de la caroube soluble à froid présentant une solubilité en milieu aqueux supérieure à 60% à une température comprise entre 10°C et 45°C dont la concentration est comprise entre 0.2 et 0.75% en poids et au moins deux pectines de natures différentes dont 0.1 à 5% en poids de pectines hautement estérifiées, et 1 à 8% en poids de pectine faiblement estérifiées, lesdits pourcentages étant exprimés en poids par rapport au poids total de composition.

Les compositions décrites par les inventeurs, notamment les compositions anti-régurgitations (et/ou anti-reflux), présentent avantageusement une viscosité d'au moins 150 mPa.s (soit au moins 150 centipoises ou 150 cP), de préférence d'au moins 300 mPa.s, encore plus préférentiellement d'au moins 600 mPa.s, dès pH=6, à une température comprise entre 35 et 40°C, typiquement d'environ 37°C.

Ces compositions permettent de réduire ou d'éviter au moins un, de préférence au moins deux, encore plus préférentiellement l'ensemble des troubles intestinaux suivants : constipation, selles molles, selles liquides et/ou trop fréquentes (diarrhée), coliques.

Avantageusement, la viscosité est évaluée grâce à un viscosimètre type Brookfield (DV-I Prime) à une température comprise entre 35 et 40°C, typiquement d'environ 37°C, avec un module S61 en forme de cylindre ou S62 en forme de disque et des vitesses de rotation variant de 12 à 100, par exemple de 20 à 100, rotations par minute (rpm) en fonction des valeurs de viscosité mesurées.

Il est également possible de mesurer le temps d'écoulement d'un volume constant de produit à travers un orifice calibré.

Typiquement, pour des valeurs de viscosité supérieures à 0 mPa.s et inférieures à 100 mPa.s la viscosité est évaluée avec un mobile S61 à 60 rpm (rotations par minute). Pour des valeurs de viscosité supérieures ou égales à 100 mPa.s et inférieures à 500 mPa.s, la viscosité est évaluée avec un mobile S62 à 60 rpm. Pour des valeurs de viscosité supérieures ou égales à 500 mPa.s et inférieures à 999.8 mPa.s, la viscosité est évaluée avec un mobile S62 à 30 rpm. Pour des valeurs de viscosité supérieures ou égales à 998 mPa.s et inférieures à 2500 mPa.s, la viscosité est évaluée avec un mobile S62 à 12 rpm.

Dans la composition selon l'invention, la concentration en caroube soluble à froid est comprise entre 0.2 et 0.75% en poids, par exemple 0.5% en poids, par rapport au poids total de composition. Tous les pourcentages en poids mentionnés dans la présente description sont exprimés en référence au poids total de la composition dans laquelle ils sont présents.

Les pectines utilisées dans la composition sont de natures différentes et sont un mélange de pectines hautement estérifiées et de pectines faiblement estérifiées, typiquement de pectines hautement estérifiées et de pectines amidées et faiblement estérifiées.

Dans un mode de réalisation particulier, la composition comprend de la caroube soluble à froid, présentant une solubilité en milieu aqueux supérieure à 60% à une température comprise entre 10°C et 45°C dont la concentration est comprise entre 0.2 et 0.75% en poids et au moins deux pectines de natures différentes dont 0.1 à 5% en poids de pectines hautement estérifiées, et 1 à 8% en poids de pectine faiblement estérifiées, lesdits pourcentages étant exprimés en poids par rapport au poids total de composition, et éventuellement de l'amidon, typiquement de l'amidon précuit et/ou prégélatinisé.

Dans un mode particulier de réalisation, la composition selon l'invention comprend en outre un ou plusieurs ingrédients choisi parmi des glucides, lesdits glucides étant de préférence choisis parmi le lactose, des maltodextrines ; des lipides ; des protéines, lesdites protéines étant hydrolysées ou non-hydrolysées ; et/ou des acides aminés.

Une composition particulière comprend de la caroube soluble à froid à une concentration, comprise entre 0.2% et 0.75% en poids, de manière plus préférée d'environ 0.5% ; et de préférence de l'amidon à une concentration comprise entre environ 1% et environ 10% en poids, typiquement entre environ 1% et environ 5% en poids ; les dites concentrations étant des concentrations exprimées en poids par rapport au poids total de composition.

Selon encore un autre aspect particulier, la composition précédente comprend en outre du lactose à une concentration comprise entre environ 20 et environ 25% en poids, et/ou des maltodextrines à une concentration comprise entre environ 15 et environ 30% en poids.

La composition objet de l'invention peut se présenter aussi bien sous forme de poudre que sous forme liquide. Elle se présente avantageusement sous la forme d'une poudre pasteurisée ou d'un liquide stérile.

Un objet particulier décrit par les inventeurs concerne ainsi une composition pour une utilisation dans un lait infantile, une supplémentation alimentaire pour nourrisson, un lait de suite, un lait de croissance, ou un aliment diététique, typiquement un aliment diététique pour nourrisson ou pour enfant.

Une composition particulière décrite est un aliment diététique destiné à des fins médicales spéciales, en particulier un lait infantile, typiquement un lait infantile anti-régurgitation et/ou anti-reflux, de préférence permettant de préserver (n'altérant pas) le transit intestinal du nourrisson ou de l'enfant, voire de restaurer le transit intestinal chez le nourrisson ou l'enfant souffrant de désagréments associés à une altération dudit transit. La base liquide utilisée pour sa préparation est typiquement une base liquide d'aliment diététique destiné à des fins médicales spéciales, en particulier une base liquide de lait infantile.

Les compositions décrites sont particulièrement adaptées pour une utilisation chez le nourrisson ou l'enfant auquel est administré un inhibiteur de pompes à protons (IPP) par exemple choisi parmi l'oméprazole, l'ésoméprazole, le lansoprazole, le pantoprazol et le rabéprazole.

Un autre objet de l'invention concerne une méthode de préparation d'une composition nutritionnelle d'intérêt, typiquement d'une composition nutritionnelle anti-régurgitation et/ou anti-reflux, pour nourrisson ou pour enfant se présentant sous forme de poudre, laquelle de préférence est une composition n'altérant pas le transit intestinal ou permettant de le préserver ou de le restaurer. Cette méthode comprend les étapes de :
a) préparation d'une base liquide de composition dont la teneur en matière sèche est d'au moins 20% en poids par rapport au poids total de ladite composition, par mélange, sous agitation, à une température d'au moins 60°C, des éléments constitutifs de ladite composition, lesdits éléments comprenant au moins deux pectines de nature différentes, dont 0.1 à 5% en poids de pectines hautement estérifiées, et de 1 à 8% en poids de pectines faiblement estérifiées, et de la caroube soluble à froid présentant une solubilité en milieu aqueux supérieure à 60% à une température comprise entre 10°C et 45°C, la concentration en caroube soluble à froid étant comprise entre 0.2 et 0.75%
   en poids par rapport au poids total de ladite composition, lesdits pourcentages étant exprimés par rapport au poids total de ladite composition,
b) homogénéisation de la base liquide obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) séchage par atomisation du mélange obtenu à l'issue de l'étape b), et
d) récupération de la composition obtenue à l'issue de l'étape c) sous forme de poudre.

La composition, par exemple la composition anti-régurgitation et/ou anti-reflux, ainsi obtenue est typiquement un aliment diététique destiné à des fins médicales spéciales, en particulier un lait infantile, par exemple un lait infantile anti-régurgitation et/ou anti-reflux, lequel de préférence n'altère pas le transit intestinal, permet de le préserver ou de le restaurer lorsqu'il est altéré, dont la base liquide utilisée pour la préparer est typiquement une base liquide d'aliment diététique destiné à des fins médicales spéciales, en particulier une base liquide de lait infantile.

Les éléments constitutifs d'une composition décrite comprennent en outre de l'amidon précuit et/ou prégélatinisé.

Dans un autre mode de réalisation particulier, la teneur en matière sèche de la base liquide est d'au moins 35% en poids par rapport au poids total de base liquide.

Dans un mode de réalisation particulièrement préféré, la méthode de préparation comprend en outre une étape d'application, à la base liquide obtenue à l'issue de l'étape a) ou à l'issue de l'étape b), d'un traitement thermique à une température comprise entre 60°C et 110°C pendant une durée suffisante pour pasteuriser ladite base, et permettant la récupération d'une composition anti-régurgitation et/ou anti-reflux pasteurisée sous forme de poudre à l'issue de l'étape d).

Selon un aspect décrit, la méthode de préparation comprend une étape de préparation de la base liquide réalisée sous agitation à une température comprise entre environ 60°C et environ 90°C, et maintenue sous agitation jusqu'à l'étape d'homogénéisation.

Selon encore un autre aspect, l'étape i) d'homogénéisation est avantageusement réalisée sous une pression comprise entre environ 170 bars et environ 200 bars et l'étape ii) d'homogénéisation est avantageusement réalisée sous une pression comprise entre environ 30 bars et environ 40 bars.

Un autre objet décrit par les inventeurs concerne une composition, en particulier une composition anti-régurgitation et/ou anti-reflux, caractérisée en ce qu'elle préserve le transit intestinal du nourrisson ou de l'enfant pour une utilisation combinée dans le traitement de la régurgitation et la prévention des anomalies ou troubles du transit intestinal (constipation, selles molles, diarrhées et/ou coliques, de préférence selles dures, selles molles ou liquides et/ou trop fréquentes) du nourrisson ou de l'enfant ou pour une utilisation dans la prévention des anomalies ou troubles du transit intestinal du nourrisson ou de l'enfant.

Les inventeurs décrivent également une composition, typiquement une composition anti-régurgitation et/ou anti-reflux, caractérisée en ce que ladite composition restaure le transit intestinal du nourrisson ou de l'enfant pour une utilisation combinée dans le traitement ou la prévention de la régurgitation et/ou du reflux, ainsi que des anomalies ou troubles du transit intestinal [constipation, selles molles, diarrhées et/ou coliques, de préférence selles dures, selles molles ou liquides et/ou trop fréquentes] du nourrisson ou de l'enfant.

### DESCRIPTION DETAILLEE DE L'INVENTION

Les compositions nutritionnelles, comme par exemple les laits infantiles anti régurgitation, comportant de la caroube et/ou de l'amidon en tant qu'agent(s) épaississant(s) présentent des effets secondaires désagréables sur le transit intestinal, en particulier des nourrissons (de la naissance à l'âge d'un an), dont le système digestif fragile et immature les rend particulièrement sensibles, ainsi que des enfants en bas âge (enfant de trois ans ou moins).

A l'heure actuelle, il n'existe pas de lait infantile, typiquement ayant un effet anti régurgitation efficace, qui n'altère pas le transit intestinal des sujets qui l'ingèrent, en particulier en causant des diarrhées, typiquement celui des sujets préidentifiés.

L'objet de l'invention est précisément de répondre à ce besoin en décrivant une composition, typiquement une composition nutritionnelle anti-régurgitation et/ou anti-reflux, qui limite l'altération, de préférence n'altère pas, de manière encore plus préférée préserve, voire restaure, le transit intestinal du sujet auquel elle est administrée. L'objet de l'invention est une composition nutritionnelle, en particulier une composition nutritionnelle anti-régurgitation et/ou anti-reflux pour nourrisson ou enfant, laquelle de préférence altère moins que les compositions de l'art antérieur, idéalement n'altère pas, le transit intestinal dudit nourrisson ou dudit enfant, ou en d'autres termes permet de préserver son transit intestinal et donc de limiter ou réduire, idéalement d'éviter, la constipation, les selles molles, les selles liquides, les selles trop fréquentes et/ou les coliques, de préférence la constipation, les selles molles, les selles liquides et/ou trop fréquentes.

Dans le cadre de la présente invention, l'expression « composition anti-régurgitation » désigne une composition qui ne provoque ni régurgitation ni reflux gastro-œsophagien, de préférence qui limite les régurgitations ou le reflux gastro-œsophagien, et idéalement qui empêche ou prévient les régurgitations ou le reflux gastro-œsophagien.

Une composition qui « préserve le transit intestinal » désigne une composition qui altère moins (que les compositions connues de l'art antérieur), de préférence n'altère pas le transit intestinal, typiquement n'altère pas le transit intestinal de 90% des nourrissons ou enfants d'une population testée, typiquement qui n'entraîne pas d'effets indésirables sur les selles, telles que constipation, diarrhées, selles molles et/ou liquides et/ou selles trop fréquentes, et/ou coliques.

La consistance des selles peut s'évaluer à l'aide de différentes échelles, connues de l'homme du métier, par exemple à l'aide de l'échelle de Bristol, définie de la façon suivante :
Type 1 : Selles dures et morcelées (en billes) d'évacuation difficile
Type 2 : Selles dures, moulées en saucisse et bosselées
Type 3 : Selles dures, moulées en saucisse, à surface craquelée
Type 4 : Selles molles mais moulées, en saucisse (ou serpentin)
Type 5 : Selles molles morcelées, à bords nets et d'évacuation facile
Type 6 : Selles molles morcelées, à bords déchiquetés
Type 7 : Selles totalement liquides

Dans le contexte de la présente invention, les selles dites « dures » correspondent aux types 1, 2 et 3 de l'échelle de Bristol. Les selles dites « formées ou normales » correspondent aux types 4 et 5, les selles dites « molles » correspondent au type 6 et les selles dites « liquides » correspondent au type 7 de cette même échelle.

Le terme « diarrhée » est typiquement employé pour désigner des selles molles et/ou liquides et/ou trop fréquentes, par exemple l'émission d'au moins trois, typiquement d'au moins quatre selles molles et/ou liquides par jour, de préférence d'au moins cinq selles molles et/ou liquides par jour.

Dans le cadre de la présente invention, l'expression « restaurer le transit intestinal » désigne typiquement l'effet consistant à rétablir un transit normal lorsque ce dernier est altéré, en particulier l'effet consistant à rétablir un transit intestinal normal chez des sujets souffrant de constipation, selles molles, selles liquides, selles molles à liquides, selles trop fréquentes et/ou coliques, de préférence chez des sujets souffrant de constipation, diarrhée, selles dures, molles et/ou selles liquides. Il s'agit donc en particulier de normaliser la consistance des selles chez des sujets dont les selles sont dures, molles ou liquides, de préférence de façon à obtenir des selles formées.

La composition objet de l'invention comprend de la caroube soluble à froid et plusieurs pectines, de préférence au moins deux pectines de natures différentes, comme indiquée dans les revendications.

La gomme de caroube native est une caroube qui présente une solubilité faible en milieu aqueux de l'ordre de 20% à une température comprise entre 10°C et 45°C (cf. demande de brevet FR 2 913 857). L'expression "solubilité en milieu aqueux à une température comprise entre 10°C et 45°C" signifie qu'à une température comprise entre 10°C et 45°C, en milieu aqueux, la gomme de caroube développe au moins 20% de la viscosité qu'elle aurait développée si elle avait été mise en solution à des températures supérieures à 80°C. Cette forme de caroube ne présente ainsi pas, à la température de reconstitution du lait dans le biberon (qui se situe entre environ 30°C et 50°C), une solubilité satisfaisante en milieu aqueux. Une telle solubilité étant cependant nécessaire et préalable à toute augmentation homogène de viscosité, on a cherché à développer des caroubes dites «solubles à froid» dont une proportion importante du potentiel visqueux s'exprime déjà entre 30 et 50°C. La gomme de caroube "soluble à froid" est au contraire une caroube qui présente une solubilité en milieu aqueux supérieure à 60% à une température comprise entre 10°C et 45°C selon la définition donnée ci-dessus.

La caroube dite « soluble à froid » se distingue de la caroube native, notamment en ce qu'elle possède une masse moléculaire moyenne en poids de caroube inférieure à celle de la caroube native. Cette masse moléculaire moyenne en poids particulière peut, par exemple, être obtenue par la rupture des liaisons glycosidiques dans le but de produire des chaînes plus courtes. Une telle caroube soluble à froid peut par exemple s'obtenir par le procédé suivant :
(i) on hydrate l'endosperme de la gomme de caroube native ;
(ii) on procède à un séchage et un broyage simultanés de l'endosperme hydraté ;
(iii) à l'issue de l'étape (ii) on réduit la masse moléculaire moyenne en poids de la caroube par une réaction de dépolymérisation de celle-ci.

La dépolymérisation de la caroube peut, par exemple, s'effectuer pas oxydation, par voie enzymatique, par hydrolyse acide, sous l'effet de pressions et de températures élevées et en présence d'un oxydant, ou par traitements physiques comme par exemple par exposition à des rayonnements de type gamma.

La caroube soluble à froid selon l'invention présente avantageusement une masse moléculaire moyenne en poids (*M̅_{w}*) comprise entre 2,5.10⁵ et 1,5.10⁶ g/mol, de préférence entre 2,5.10⁵ et 1.10⁶ g/mol, de préférence encore entre 2,5.10⁵ et 6.10⁵ g/mol.

Les caroubes « solubles à froid » sont connues pour provoquer une augmentation rapide de la viscosité de la base de lait liquide, de sorte que dans l'esprit de l'homme du métier, l'application de certains traitements technologiques, tels que l'application d'un traitement thermique (lors de l'étape de pasteurisation ou stérilisation), l'homogénéisation et l'atomisation (pulvérisation) nécessaires à la production de poudre alimentaire, en particulier de laits infantiles en poudre pasteurisée, sont considérés par l'homme du métier comme impossibles à mettre en œuvre en présence de caroube. Jusqu'ici la caroube soluble à froid n'était donc utilisée qu'en mélange à sec et non liquide ce qui présentait l'inconvénient de ne pas permettre l'élimination systématique des bactéries pathogènes susceptibles de contaminer la composition, ladite élimination étant uniquement permise par la mise en œuvre d'une étape de stérilisation ou de pasteurisation appliquée à un mélange liquide.

A noter que pour le lait, en particulier le lait infantile, la flore totale maximale tolérée ne doit en effet de préférence pas excéder 1000 CFU (colonies formant unité) par gramme de poudre. Parmi cette flore, Clostridium perfringens, Escherichia coli, Bacillus cereus, Listeria monocytogenes, les Staphylocoques à coagulase +, les salmonelles et les entérobactéries (notamment Cronobacter sakazaki) sont en particulier considérées comme pathogènes pour l'être humain et doivent de préférence être totalement éliminés. Le procédé selon l'invention permet à présent d'éliminer l'ensemble des bactéries pathogènes (en particulier les bactéries listées précédemment) pour l'être humain, en particulier pour le nourrisson et l'enfant, notamment l'enfant en bas âge.

Les inventeurs ont mis au point et décrivent à présent une composition, en particulier une composition anti régurgitation et/ou anti reflux, ayant de manière surprenante un effet anti-régurgitation et/ou anti-reflux très efficace, ladite composition comprenant de la caroube soluble à froid et de préférence des pectines, encore plus préférentiellement de la caroube soluble à froid et au moins deux pectines de natures différentes, et ladite composition étant obtenue à l'aide d'un procédé qui permet de surmonter les contraintes techniques connues de l'homme du métier, tout en permettant un net progrès en terme de sécurité alimentaire.

La composition objet de l'invention comporte en effet de la caroube soluble à froid. La composition comprend de la caroube soluble à froid dans une concentration comprise entre environ 0.2% et environ 0.75 % en poids par rapport au poids total de composition, de préférence environ 0.5% en poids.

La composition objet de l'invention comporte au moins deux pectines de natures différentes. Dans le contexte de la présente invention, les termes « pectine » et « substance pectique » sont indifféremment employés. Les substances pectiques sont des polymères de polysaccharides acides. Elles sont composées d'une chaîne principale constituée de monomères d'acide uronique liés en 1-4 entre lesquels s'intercalent des molécules de rhamnoses par des liaisons 1-2 et 1-4 responsables de la forme de zig-zag des macromolécules de pectine. Ces molécules complexes présentent des ramifications au niveau des acides uroniques comme au niveau du rhamnose par des molécules de type galactanes, rhamnanes, etc.

Il existe une grande variété de pectines dont l'origine est exclusivement végétale. Les pectines sont présentes en grande quantité dans les pépins et les zestes de groseilles, pommes, coings et agrumes.

Les pectines peuvent former un réseau ayant ainsi un effet gélifiant. Ce sont des composés très fréquemment utilisés dans les préparations nutritionnelles comme les confitures, justement pour ces propriétés gélifiantes.

Les pectines sont connues pour être des stabilisateurs ou épaississants (en augmentant la viscosité) de compositions nutritionnelles et notamment de laits infantiles (US2003/0165606).

Elles sont également utilisées pour lutter contre les diarrhées (Howard P, Pectin-Agar for Diarrhea in infants and the newborn. JAMA, 1940, 114(24) : 2355-2358).

Un médicament, connu depuis plusieurs décennies, vendu en pharmacie sous l'appellation Gelopectose®, est par ailleurs utilisé pour épaissir le contenu de l'estomac.

Comme indiqué précédemment, les pectines utilisées dans la composition selon l'invention sont de natures différentes. Les pectines utilisées de natures différentes sont un mélange de pectines hautement estérifiées et de pectines faiblement estérifiées, typiquement de pectines amidées et faiblement estérifiées.

Les pectines « hautement estérifiées » sont typiquement des pectines hautement méthylées (encore identifiées dans le présent texte en tant que pectines « HM »).

Les pectines « faiblement estérifiées » sont typiquement des pectines faiblement méthylées (encore identifiées dans le présent texte en tant que pectines « LM »).

Les pectines sont des polymères complexes présents dans les membranes cellulaires des plantes. Les pectines sont composées majoritairement d'acide galacturonique, dont le groupe carboxyl est fréquemment méthylé. D'autres sucres tels que le rhamnose, le galactose et l'arabinose peuvent aussi être présents, selon la plante source et la méthode d'extraction.

Les pectines « faiblement estérifiées » utilisées dans le cadre de la présente invention sont typiquement des pectines d'agrumes.

La composition présente à la fois des pectines faiblement estérifiées (de préférence faiblement méthylées) et des pectines hautement estérifiées (de préférence hautement méthylées). Dans un mode de réalisation encore plus préféré, les pectines faiblement estérifiées sont amidées.

Le degré d'amidation de la « pectine amidée et faiblement estérifiée », présente dans la composition selon l'invention, est compris entre environ 5% et environ 30%, de préférence entre environ 5% et environ 20%, plus préférentiellement entre environ 10% et environ 20%. Le degré d'amidation désigne le pourcentage d'acides galacturoniques portant un groupement amide au sein d'une molécule de pectine.

Son degré d'estérification, typiquement de méthylation, est typiquement compris entre environ 20% et environ 50%, préférentiellement entre environ 30% et environ 50%, et plus préférentiellement entre environ 30% et environ 40%. Le degré d'estérification désigne le pourcentage d'acides galacturoniques méthyl-estérifiés au sein d'une molécule de pectine.

Lorsque la composition décrite est par exemple un lait infantile, la présence, au sein de cette composition, d'au moins une pectine faiblement estérifiée, de préférence d'au moins une pectine amidée et faiblement estérifiée, permet d'obtenir un lait infantile reconstitué (après la dissolution de la poudre dans l'eau) présentant une faible viscosité, typiquement un lait liquide présentant une viscosité comprise entre 20 et 50 mPa.s à un pH de reconstitution.

Un lait reconstitué à partir d'un lait sous forme de poudre décrit par les inventeurs typiquement d'un lait anti-régurgitation sous forme de poudre selon l'invention, présente une viscosité particulièrement adaptée, dans l'estomac, dès pH=6, et plus généralement à un pH compris entre 6 et 5, par exemple entre 5,8 et 5,5, typiquement à un pH de 5,2, 5,3, 5,4, 5,5, 5,6 et 5,7.

Par ailleurs, le pH requis pour le développement d'une viscosité satisfaisante au sens de l'invention est significativement supérieur pour la composition selon l'invention au pH requis, d'environ 3,5 pour les compositions anti-régurgitation classiques. Ainsi, le pH gastrique diminuant progressivement, après un repas, de 7 à 3 environ, le produit selon l'invention développe une viscosité plus rapidement dans l'estomac que les laits anti-régurgitation classiques et permet ainsi de limiter voire d'éliminer les symptômes de régurgitation ou de reflux lorsqu'ils sont présents. Il est en outre facile à boire par la tétine du biberon et permet au sujet de s'alimenter sans frustration tout en limitant les risques d'aérophagie.

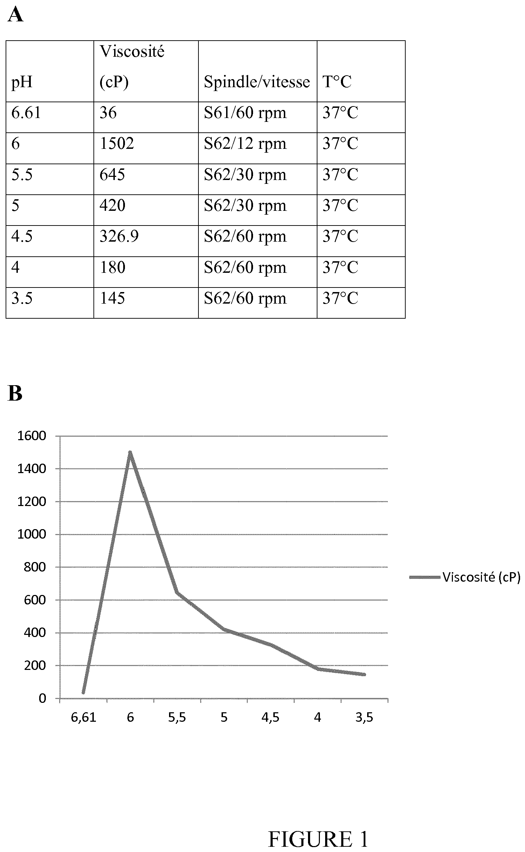

La viscosité optimale souhaitée du lait infantile décrit par les inventeurs, en particulier du lait infantile anti-régurgitation et/ou anti-reflux décrit est de préférence inférieure à environ 50 mPa.s mesurée à un pH de reconstitution (i.e. un pH d'environ 7) et à une température comprise entre 35°C et 40°C, et supérieure à environ 150 mPa.s, de préférence à environ 500 mPa.s, encore plus préférentiellement comprise entre environ 500 et environ 600 mPa.s, lorsqu'elle est mesurée à pH= 5,5, et à une température également comprise entre environ 35°C et environ 40°C (cf. Figure 1).

La viscosité optimale souhaitée du lait infantile décrit par les inventeurs, en particulier du lait infantile anti-régurgitation et/ou anti-reflux décrit est de préférence inférieure à 50 mPa.s mesurée à un pH de reconstitution (d'environ 7) et à une température comprise entre environ 35°C et environ 40°C, et supérieure à environ 150 mPa.s, de préférence à environ 600 mPa.s, encore plus préférentiellement comprise entre environ 800 et environ 1600 mPa.s, par exemple 1500 mPa.s, lorsqu'elle est mesurée à pH= 6, et à une température également comprise entre 35°C et 40°C (cf. Figure 1).

Dans les compositions décrites, typiquement dans les laits infantiles anti-régurgitation et/ou anti-reflux, les pectines, typiquement les « au moins deux pectines de natures différentes » sont présentes à une concentration inférieure ou égale à environ 10% en poids par rapport au poids total de composition, typiquement comprise entre environ 4% et environ 10% en poids par rapport au poids total de composition, par exemple environ 5% en poids par rapport au poids total de composition.

Dans les compositions selon l'invention, typiquement dans les laits infantiles anti-régurgitation et/ou anti-reflux, la « pectine faiblement estérifiée » est présente à une concentration comprise entre 1% et 8%, par exemple entre environ 3% et environ 8%, de manière encore plus préférée entre environ 3% et environ 5%, et est typiquement de environ 4%.

Avantageusement, la concentration, au sein d'une composition décrite par les inventeurs, de pectine faiblement estérifiée et éventuellement de pectine fortement estérifiée sera adaptée à la nature et à la quantité des protéines éventuellement présentes dans la composition.

Avantageusement, la concentration, au sein d'une composition décrite par les inventeurs, de pectine faiblement estérifiée, en particulier de pectine amidée et faiblement estérifiée, sera d'autant plus élevée que ladite composition sera riche en protéines hydrolysées et, inversement, cette concentration sera d'autant plus basse que ladite composition sera riche en protéines non hydrolysées.

Les pectines « hautement estérifiées » utilisables dans le cadre de la présente invention sont typiquement des pectines d'agrumes, en particulier des pectines d'agrumes non amidées.

Les inventeurs ont découvert qu'une telle « pectine hautement estérifiée », utilisée en combinaison avec au moins une « pectine faiblement estérifiée », de préférence avec au moins une « pectine amidée et faiblement estérifiée », permet d'améliorer la stabilité de la composition selon l'invention, typiquement de la composition anti-régurgitation et/ou anti-reflux selon l'invention, (en particulier la stabilité des protéines) à pH acide.

Le degré d'estérification, typiquement de méthylation, de la « pectine hautement estérifiée » présente dans la composition selon l'invention est compris entre environ 50% et environ 90%, de préférence entre environ 50% et environ 80%, plus préférentiellement entre environ 60% et environ 70%.

L'indice de stabilité de ladite pectine à pH = 4 est compris entre 140-200, de préférence entre environ 150 et environ 190, plus préférentiellement entre environ 165 et environ 185. Cet indice, compris entre 100 et 200, est une mesure par sédimentation de la capacité de la pectine hautement estérifiée à protéger les protéines non-hydrolysées en milieu acide de la floculation et/ou coagulation.

Dans les compositions nutritionnelles selon l'invention, typiquement dans les compositions anti-régurgitation et/ou anti-reflux, en particulier préservant le transit intestinal ou permettant de le restaurer, la « pectine hautement estérifiée » est présente à une concentration comprise entre 0.1% et 5%, plus préférentiellement d'environ 1%.

De manière préférée, la concentration de pectine hautement estérifiée, au sein d'une composition décrite comprenant au moins une pectine faiblement estérifiée, de préférence au moins une pectine amidée et faiblement estérifiée, et au moins une pectine hautement estérifiée, sera d'autant plus élevée que ladite composition sera riche en protéines non hydrolysées et, inversement, cette concentration sera d'autant plus basse que ladite composition sera riche en protéines hydrolysées et/ou acides aminés en mélange.

Dans une composition particulière décrite par les inventeurs la concentration de protéines non hydrolysées est comprise entre environ 10 et 13 % en poids par rapport au poids total de composition, celle de pectine faiblement estérifiée, typiquement celle de pectine amidée et faiblement estérifiée, est comprise entre 4 et 5% (elle est par exemple de 4.5%) et celle de pectine hautement estérifiée est d'environ 1%.

Dans une composition particulière décrite par les inventeurs, la concentration de protéines hydrolysées est comprise entre environ 10% et environ 13% en poids, celle de pectine faiblement estérifiée, typiquement celle de pectine amidée et faiblement estérifiée, est d'environ 5% en poids, et celle de pectine hautement estérifiée est d'environ 1% en poids par rapport au poids total de composition.

Dans une composition particulière décrite par les inventeurs la concentration de protéines non hydrolysées est comprise entre environ 8% et environ 10% en poids (elle est typiquement d'environ 9% en poids), la concentration de protéines hydrolysées est comprise entre environ 2% et environ 4% en poids (elle est typiquement d'environ 3% en poids), celle de pectine faiblement estérifiée, typiquement celle de pectine amidée et faiblement estérifiée, est comprise entre environ 4 et environ 4.5% en poids (elle est par exemple de 4.25%), et celle de pectine hautement estérifiée est d'environ 1% en poids par rapport au poids total de composition.

La composition objet de l'invention peut également comprendre de l'amidon.

L'amidon est composé d'amylose, polymère linéaire de glucose lié par des liaisons α 1-4 et d'amylopectine, polymère ramifié de glucose contenant à la fois des liaisons α 1-4 prédominantes et des ramifications α 1-6. Selon la source végétale dont il est extrait, l'amidon a une composition un peu différente en amylose et en amylopectine.

L'alpha-amylase salivaire n'est pas fonctionnelle à la naissance. Son activité à l'âge de 3 mois est nettement inférieure à celle de l'adulte. De même, l'activité de cette enzyme au niveau pancréatique augmente progressivement jusqu'à l'âge de 3 ans. Toutefois, la gluco-amylase qui intervient peu chez l'adulte dans la digestion de l'amidon est très active dès la naissance. Cette répartition différente des activités enzymatiques permet au nouveau-né de digérer jusqu'à 3 g/kg/j d'amidon. L'amidon a pour caractéristique de voir sa structure se modifier sous l'effet de la chaleur et de l'hydratation. On distingue différentes formes d'amidons tels que l'empois ou le gel d'amidon dans lesquels la solubilisation conduit à une augmentation de la viscosité et à une amélioration de la digestibilité. Ces faits justifient son utilisation uniquement sous forme d'amidon pré-cuit ou pré-gelatinisé dans les laits anti-régurgitations. Par ailleurs, la digestibilité de l'amidon est meilleure lorsque celui-ci comporte une plus grande quantité d'amylopectine.

Les amidons, en association avec des protéines, les caséines en particulier, qui floculent à pH acide, développent, au niveau de l'estomac, la viscosité recherchée du bol alimentaire qui permet ainsi de réduire ou d'éviter les régurgitations et reflux par effet de pesanteur.

Selon un aspect, la composition nutritionnelle décrite comporte de l'amidon à une concentration inférieure ou égale à 5% en poids par rapport au poids total de composition. D'une manière préférée, la composition comporte de l'amidon dans une concentration inférieure ou égale à 2.5% en poids par rapport au poids total de composition. D'une manière également préférée, la composition comporte de l'amidon dans une concentration inférieure ou égale à 1% en poids par rapport au poids total de composition. Dans un autre mode de réalisation préféré entre tous, la composition décrite ne comprend pas d'amidon.

Un objet décrit est donc une composition nutritionnelle, typiquement une composition anti-régurgitation et/ou anti-reflux, en particulier préservant le transit intestinal ou permettant de restaurer le transit intestinal, comprenant en pourcentages de poids par rapport au poids total de composition: 0.2-0.75% de caroube soluble à froid, encore plus préférentiellement environ 0.5% de caroube soluble à froid ; entre environ 4% et environ 5% de pectines amidées faiblement estérifiées ; et environ 1% de pectines hautement estérifiées. Selon un aspect particulier, la composition décrite comprend en outre de l'amidon, de préférence de l'amidon précuit et/ou prégélatinisé.

Ainsi, selon un autre aspect, la composition nutritionnelle, typiquement anti-régurgitation et/ou anti-reflux, en particulier préservant le transit intestinal ou permettant de le restaurer, comprend en pourcentages de poids par rapport au poids total de composition: 0.2-0.75% de caroube soluble à froid, encore plus préférentiellement environ 0.5% de caroube soluble à froid ; entre environ 4% et environ 5% de pectines amidées faiblement estérifiées (typiquement faiblement méthylées) ; environ 1% de pectines hautement estérifiées (typiquement hautement méthylées), et éventuellement entre environ 0.5% et environ 5% d'amidon, de préférence environ 1% d'amidon.

Selon un autre aspect, la composition nutritionnelle décrite, typiquement la composition anti-régurgitation et/ou anti-reflux, en particulier préservant le transit intestinal ou permettant de restaurer le transit intestinal, comprend en outre environ 26% de maltodextrines et environ 22% de lactose, exprimés en poids par rapport au poids total de composition.

Un objet particulier décrit par les inventeurs est un lait infantile, par exemple un lait anti-régurgitation et/ou anti-reflux, qui n'altère pas le transit intestinal du nourrisson ou de l'enfant et n'entraîne donc pas d'effets sur les selles ou permettant de restaurer le transit intestinal. Ce lait infantile est obtenu à partir de la composition décrite précédemment comprenant de la caroube soluble à froid et des pectines, typiquement au moins deux pectines de natures différentes.

Un lait infantile, typiquement anti-régurgitation et/ou anti-reflux, tel que décrit dans le présent texte, peut comprendre toute base de lait infantile connue de l'homme du métier. Ainsi, n'importe quelle base de lait infantile, dont les propriétés nutritionnelles sont adaptées au besoin des nourrissons et enfants, y compris les Aliments Diététiques Destinés à des Fins Médicales Spéciales (ADDFMS), peut être utilisée pour préparer une composition telle que décrite par les inventeurs.

Une base de lait infantile standard comprend des glucides, des lipides, des protéines, des minéraux, des vitamines et éventuellement des facteurs de croissance. Les proportions habituelles de ces différents constituants au sein de la base de lait sont d'environ 55% pour les glucides, 25% pour les lipides, 15% pour les protéines et 5% pour l'ensemble constitué par les minéraux et vitamines, les pourcentages étant calculés par rapport au poids total (ou à la masse totale) de matière sèche de la base de lait déshydratée.

La base de lait peut en outre éventuellement comprendre d'autres composés connus de l'homme du métier tels que les composés améliorant la texture du lait, le goût du lait et/ou présentant un intérêt nutritionnel ou fonctionnel spécifique (nucléotides, probiotiques, prébiotiques, etc.).

Classiquement, la fraction protéique de la base de lait infantile peut comprendre deux types de protéines : des protéines d'origine animale, notamment celles issues du lait (caséine et/ou protéines solubles, encore appelées protéines du lactosérum), et des protéines d'origine végétale. La fraction protéique peut toutefois avantageusement comprendre l'un seulement de ces deux types de protéines, par exemple uniquement des protéines végétales.

Les protéines d'origine animale peuvent provenir par exemple de lait de vache, de lait de chèvre, de lait humain, de lait de chamelle, de lait de bufflonne, lait d'ânesse et/ou de lait de jument.

Les protéines d'origine végétale peuvent provenir par exemple du riz, du soja, du pois, du maïs, du blé et/ou de la pomme de terre.

Les protéines présentes dans la base de lait infantile, utilisée dans le cadre de la présente invention, peuvent être entières ou, au contraire, totalement ou partiellement hydrolysées. Il peut également s'agir d'un mélange des deux types de protéines, par exemple comprenant 75% de protéines entières pour 25% de protéines hydrolysées. Les protéines hydrolysées ont de préférence un degré d'hydrolyse compris entre environ 5% et environ 90%, de préférence entre environ 5% et environ 50%. Le degré d'hydrolyse correspond au nombre de liaisons peptidiques rompues par l'hydrolyse. Plus ce nombre est élevé, plus l'hydrolyse est poussée.

Selon des aspects particuliers décrits dans le contexte de la présente invention, le degré d'hydrolyse des protéines hydrolysées est compris entre environ 5% et environ 50%, par exemple entre 30% et 40%, entre 5% et 20%, entre 5% et 10%, par exemple 7%.

La fraction protéique peut également comprendre des acides aminés en mélange. Les acides aminés en mélange peuvent être des acides aminés naturels, des acides aminés de synthèse ou un mélange d'acides aminés naturels et d'acides aminés de synthèse.

Les acides aminés en mélange peuvent constituer à eux seuls la fraction protéique du lait infantile anti-régurgitation et/ou anti-reflux selon la présente invention. Ils peuvent également être présents à côté des protéines hydrolysées et des éventuelles protéines non hydrolysées.

Ces protéines hydrolysées et/ou acides aminés en mélange sont plus digestes que les protéines non hydrolysées et permettent d'accélérer la vidange gastrique. Une composition décrite préférée, en particulier une composition anti-régurgitation n'ayant pas d'effet sur les selles préférée, comprend des protéines hydrolysées et des acides aminés en mélange, par exemple environ 95% de protéines hydrolysées pour 5% d'acides aminés en mélange.

Ainsi une composition particulière décrite par les inventeurs, typiquement une composition anti-régurgitation et/ou anti-reflux, en particulier n'altérant pas le transit du nourrisson ou de l'enfant ou permettant de restaurer le transit intestinal, comprend une fraction protéique contenant une majorité de protéines hydrolysées et/ou d'acides aminés en mélange.

Une autre composition particulière décrite par les inventeurs, typiquement une autre composition anti-régurgitation et/ou anti-reflux, en particulier n'altérant pas le transit du nourrisson ou de l'enfant ou permettant de restaurer le transit intestinal, comprend une fraction protéique contenant une majorité de protéines non hydrolysées.

Les lipides typiquement susceptibles de rentrer dans la composition du lait infantile décrite peuvent être choisis par exemple parmi la matière grasse du lait, l'huile de carthame, les lipides de jaune d'œuf, l'huile d'olive, l'huile de noix de coco, l'huile de palme, l'huile de soja, l'huile de tournesol, l'huile de poisson, les huiles issues d'algues et/ou de champignons, l'oléine de palme, les triglycérides à chaînes moyennes, et les esters d'acides gras choisis, par exemple, parmi l'acide arachidonique, l'acide linoléique, l'acide palmitique, l'acide stéarique, l'acide docosahexanoïque, l'acide eicosapentanoique, l'acide linolénique, l'acide oléique, l'acide laurique, l'acide caprique, l'acide caprylique et l'acide caproïque.

Les glucides susceptibles de rentrer dans la composition du présent lait infantile (autres que les agents épaississants décrits plus tard dans la présente description) peuvent être n'importe quel ose ou oside connu de l'homme de l'art comme étant adapté à la nutrition humaine, typiquement infantile. Typiquement, les glucides peuvent être choisis parmi le lactose, les maltodextrines ou sirop de glucose, le saccharose, le fructose, et le glucose.

Des exemples de sels minéraux, de vitamines et d'autres nutriments éventuellement présents dans le lait infantile anti-régurgitation décrit incluent la vitamine A, la vitamine B6, la vitamine B12, la vitamine D, en particulier la vitamine D3 (cholécalciférol), la vitamine E, la vitamine K, la vitamine C, l'acide folique, la thiamine, l'inositol, la riboflavine, la niacine, la biotine, l'acide pantothénique, la choline, le calcium, le phosphore, l'iode, le fer, le magnésium, le cuivre, le zinc, le manganèse, le chlore, le potassium, le sodium, le sélénium, le chrome, le molybdène, la taurine, et la L-Carnitine.

En plus des considérations de compatibilité et de stabilité liées aux procédés de préparation décrits dans le cadre de la présente invention et aux conditions de stockage d'une composition, typiquement d'un lait, décrite, la présence et les quantités de sels minéraux et de vitamines spécifiques éventuellement présents pourront changer légèrement selon la population ciblée [nourrissons (typiquement de la naissance à l'âge d'un an, par exemple de la naissance à l'âge de 6 mois) ou enfants en bas âge (typiquement enfants de moins de trois ans, par exemple enfants âgés de 6 à 18 mois)].

La composition, typiquement la composition anti-régurgitation et/ou anti-reflux, en particulier n'altérant pas le transit du nourrisson ou de l'enfant ou permettant de restaurer le transit intestinal, se présente avantageusement sous la forme d'une poudre à reconstituer, i.e., à dissoudre dans une boisson (ou dans tout liquide consommable), typiquement de l'eau ou du lait, par exemple du lait de vache ou de chèvre, avant ingestion par le sujet, en particulier lorsque ledit sujet présente des symptômes de régurgitations et/ou reflux ou est susceptible de présenter des symptômes de régurgitations (i.e. la grande majorité des nourrissons et enfants en bas âge).

Un objet particulier décrit par les inventeurs concerne une boisson reconstituée telle qu'une boisson à pH neutre, par exemple un lait reconstitué à partir d'une composition décrite par les inventeurs se présentant sous forme de poudre.

Typiquement, un lait infantile liquide décrit par les inventeurs, typiquement un lait anti-régurgitation et/ou anti-reflux, en particulier n'altérant pas le transit du nourrisson ou de l'enfant ou permettant de restaurer le transit intestinal, comprend une teneur dudit lait infantile en poudre de 11 à 15 % en poids, de préférence de 13 % en poids par rapport au poids total de lait reconstitué.

Un autre objet particulier de l'invention concerne un concentré liquide (préparé à partir d'une composition sous forme de poudre décrite par les inventeurs) susceptible d'être dilué, de préférence à l'aide d'un liquide dont le pH est proche de la neutralité.

Typiquement, un lait décrit par les inventeurs, en particulier un lait anti-régurgitation et/ou anti-reflux, de préférence n'altérant pas le transit du nourrisson ou de l'enfant ou permettant de restaurer ledit transit intestinal, se présentant sous forme de concentré liquide, préparé à partir d'une composition sous forme de poudre décrite par les inventeurs, comprend une teneur (i.e., pourcentage de matière sèche) dudit lait infantile de 25 à 50 % en poids ou masse, de préférence de 35% en poids ou masse. Un tel lait peut être dilué pour obtenir un lait infantile liquide comprenant une teneur dudit lait infantile en poudre de 11 à 15 % en poids ou masse, de préférence de 13 % en poids ou en masse.

La conservation du lait infantile concentré est avantageusement assurée par une stérilisation en autoclave et préférentiellement par un traitement thermique UHT suivi d'un conditionnement aseptique.

Les laits décrits par les inventeurs peuvent être administrés par voie orale ou entérale. Ils sont préférentiellement administrés par voie orale puisqu'ils ont, à côté de leur rôle alimentaire, par exemple pour rôle de prévenir, limiter voire supprimer les symptômes de régurgitation et/ou reflux, sans altérer (tout en préservant) le transit intestinal, voire en restaurant le transit intestinal.

La composition selon l'invention présente en effet l'avantage significatif, d'être, une fois reconstituée, liquide à pH 7 et visqueuse à un pH compris entre 6 et 3,5, avantageusement dès pH=6, typiquement pour un pH compris entre 6 et 5, par exemple à pH=5. Elle peut donc être utilisée en tant que composition anti-régurgitation et/ou anti-reflux. La composition objet de l'invention préserve également avantageusement le transit intestinal du nourrisson comme expliqué dans le présent texte.

Au sens de la présente invention, la viscosité du bol alimentaire est considérée comme satisfaisante si elle permet de diminuer, idéalement d'éliminer les symptômes de régurgitations et/ou de reflux.

La viscosité peut être mesurée avec un viscosimètre type Brookfield, avec un module en forme de disque (S62) ou de cylindre (S61) et à des vitesses de rotation comprises entre 12 et 100, par exemple 20 et 100, rotations par minute. Il est également possible de mesurer le temps d'écoulement d'un volume constant de produit à travers un orifice calibré.

Avantageusement, les compositions diluées ou reconstituées décrites par les inventeurs, ont :
- à un pH compris entre 7 et 6,6, une viscosité comprise entre 20 et 50 mPa.s, par exemple entre 20 et 45 mPa.s, de préférence entre 30 et 40 mPa.s;
- à un pH inférieur à 6,5, typiquement entre 6 et 3.5, une viscosité comprise entre 1600 et 150 mPa.s, par exemple à un pH compris entre 6 et 5, une viscosité comprise entre 1600 et 400 mPa.s.

Les composés entrant dans la formulation de la composition utilisés dans le cadre de la présente invention peuvent se présenter sous forme de poudre ou sous forme de solution aqueuse. Au sens de l'invention, les expressions « milieux aqueux » ou « solution aqueuse » désignent respectivement un milieu ou une solution qui est au moins partiellement constituée d'eau.

Un lait particulier, décrit, typiquement un lait anti-régurgitation et/ou anti-reflux, en particulier n'altérant pas le transit du nourrisson ou de l'enfant ou permettant de le restaurer, se présentant sous forme de poudre, comprend avantageusement au moins 94% de matière sèche, de préférence au moins 95% de matière sèche, de manière encore plus préférée au moins 98% de matière sèche en poids par rapport au poids total de lait sous forme de poudre.

Un autre objet décrit concerne une composition, typiquement anti-régurgitation et/ou anti-reflux, en particulier un lait infantile, une supplémentation alimentaire pour nourrisson ou enfant en bas âge, un lait de suite, un lait de croissance ou un aliment diététique pour nourrisson ou enfant, caractérisée en ce qu'elle préserve le transit intestinal du nourrisson ou de l'enfant pour une utilisation combinée dans le traitement de la régurgitation et la prévention des anomalies ou troubles du transit intestinal (tels que définis précédemment) du nourrisson ou de l'enfant.

Encore un autre objet décrit concerne une composition, typiquement anti-régurgitation et/ou anti-reflux, en particulier un lait infantile, une supplémentation alimentaire pour nourrisson ou enfant en bas âge, un lait de suite, un lait de croissance ou un aliment diététique pour nourrisson ou enfant, caractérisée en ce que ladite composition restaure le transit intestinal du nourrisson ou de l'enfant pour une utilisation combinée dans le traitement de la régurgitation et/ou du reflux, ainsi que des anomalies ou troubles du transit intestinal [tels que définis précédemment] du nourrisson ou de l'enfant.

La présente description concerne également un procédé permettant l'obtention d'une composition, typiquement d'une composition anti-régurgitation et/ou anti-reflux, sous forme de poudre, en particulier d'une composition diététique ou nutritionnelle telle que décrite précédemment, de préférence d'un lait infantile, d'une supplémentation alimentaire pour nourrisson, d'un lait de suite, d'un lait de croissance ou d'un aliment diététique, de manière encore plus préférée d'un lait infantile anti-régurgitation et/ou anti-reflux n'altérant pas (ou préservant, voire restaurant) le transit intestinal du nourrisson ou de l'enfant, tel que décrit précédemment.

Ce procédé comprend les étapes suivantes de :
a) préparation d'une base liquide de composition dont la teneur en matières sèches est d'au moins 20% en poids (ou masse) par rapport au poids totale de ladite composition, par mélange, sous agitation, à une température d'au moins 60°C, des éléments constitutifs de ladite composition, lesdits éléments comprenant deux pectines de natures différentes dont 0.1 à 5% en poids de pectines hautement estérifiées, et de 1 à 8% en poids de pectines faiblement estérifiées (de manière encore plus préférée au moins une pectine faiblement méthylée et au moins une pectine hautement méthylée), et de la caroube soluble à froid présentant une solubilité en milieux aqueux supérieure à 60% à une température comprise entre 10°C et 45°C, la concentration en caroube soluble à froid étant comprise entre 0.2 et 0.75% en poids, lesdits pourcentages étant exprimés en poids par rapport au poids total de ladite composition,
b) homogénéisation de ladite base liquide obtenue à l'issue de l'étape a) par fractionnement des éléments constitutifs lors d'une première étape i) réalisée sous une pression comprise de préférence entre 100 et 300 bars et lors d'une étape ii) réalisée sous une pression comprise de préférence entre 10 et 60 bars,
c) séchage par atomisation du mélange obtenu à l'issue de l'étape b), et
d) récupération de la composition obtenue à l'issue de l'étape c) sous forme de poudre.

Le procédé précédant permet, contrairement aux procédés connus à ce jour, de préparer, de façon simple et efficace, à partir des éléments constitutifs d'une base, typiquement à partir d'une base de lait infantile se présentant sous forme liquide, une composition telle que décrite par les inventeurs, typiquement un lait infantile, qui va être séchée et transformée en poudre. La poudre obtenue à l'issue de ce procédé est homogène, et la composition liquide reconstituée à partir d'une telle poudre présente les propriétés de viscosité, décrites précédemment, requises pour limiter, idéalement supprimer, les symptômes de régurgitation et/ou de reflux, sans altérer le transit intestinal voire en l'améliorant, typiquement en le restaurant, lorsqu'il est altéré.

La composition ainsi obtenue est typiquement un aliment diététique destiné à des fins médicales spéciales, en particulier un lait infantile anti-régurgitation et/ou anti-reflux, n'altérant pas le transit intestinal, permettant de le préserver, voire idéalement de l'améliorer lorsqu'il est altéré, dont la base liquide utilisée pour la préparer est typiquement une base liquide d'aliment diététique destiné à des fins médicales spéciales, en particulier une base liquide de lait infantile.

Selon un autre aspect, les éléments constitutifs de la composition comprennent en outre de l'amidon précuit et/ou prégélatinisé. Selon encore un autre aspect particulier, la teneur en matière sèche de la base liquide est d'au moins 35% en poids par rapport au poids total de base liquide.

La composition sous forme de poudre décrite par les inventeurs peut en outre avantageusement être une composition pasteurisée (ou stérilisée).

Les compositions décrites sont particulièrement adaptées pour une utilisation chez le nourrisson ou l'enfant auquel est administré un inhibiteur de pompes à protons (IPP) par exemple choisi parmi l'oméprazole, l'ésoméprazole, le lansoprazole, le pantoprazol et le rabéprazole.

Au sens de la présente invention, la pasteurisation désigne une étape de traitement thermique provoquant la destruction des germes considérés comme pathogènes pour le sujet auquel la composition est destinée, et plus généralement une diminution de la flore bactérienne.

Classiquement le traitement thermique est réalisé à une température comprise entre environ 60°C et environ 110°C pendant une durée suffisante pour obtenir la pasteurisation, i.e. pendant une durée comprise entre environ 15 minutes et quelques secondes, par exemple environ 25 ou 30 secondes. L'homme du métier est en mesure de déterminer la durée adaptée à une température donnée qui va permettre d'obtenir la pasteurisation souhaitée sans détruire la composition, ses propriétés nutritives ou ses propriétés anti-régurgitation et/ou reflux. Concernant le lait en particulier, l'homme du métier est en mesure de déterminer les conditions adaptées à la préservation des protéines et des vitamines.

Le procédé décrit précédemment peut ainsi en outre comprendre de façon avantageuse une étape additionnelle d'application, à la base liquide obtenue à l'issue de l'étape a) ou à l'issue de l'étape b), d'un traitement thermique à une température comprise entre 60°C et 110°C pendant une durée suffisante pour pasteuriser la base liquide. Ce procédé permet ainsi l'obtention d'une composition, typiquement d'une composition anti-régurgitation et/ou anti-reflux pasteurisée sous forme de poudre n'altérant pas le transit intestinal.

Une telle composition ne présente pas les risques microbiologiques (notamment pour C. *Sakazakii*) observés lors des mélanges à sec d'une base de lait infantile avec de la poudre d'amidon ou de caroube.

Un objet particulier décrit par les inventeurs concerne ainsi un procédé ou une méthode permettant la préparation d'une composition pasteurisée sous forme de poudre, en particulier d'une composition nutritionnelle, anti-régurgitation et/ou anti-reflux pour nourrisson ou pour enfant, et/ou n'altérant pas ou permettant de restaurer le transit intestinal dudit nourrisson ou dudit enfant, en particulier d'un lait infantile ou une composition diététique tels que décrit précédemment, comprenant les étapes suivantes de :
a) préparation d'une base liquide de composition dont la teneur en matières sèches est d'au moins 20% en poids (ou en masse) par rapport au poids total de ladite composition, par mélange, sous agitation, à une température d'au moins 60°C, des éléments constitutifs de ladite composition, lesdits éléments comprenant deux pectines de natures différentes dont 0.1 à 5% en poids de pectines hautement estérifiées, et de 1 à 8% en poids de pectines faiblement estérifiées (de manière encore plus préférée au moins une pectine faiblement méthylée et au moins une pectine hautement méthylée), et de la caroube soluble à froid présentant une solubilité en milieu aqueux supérieure à 60% à une température comprise entre 10°C et 45°C, la concentration en caroube soluble à froid étant comprise entre 0.2 et 0.75% en poids, lesdits pourcentages étant exprimés en poids par rapport au poids total de ladite composition,
b) homogénéisation de la base liquide obtenue à l'issue de l'étape a) par fractionnement des éléments constitutifs lors d'une première étape i) réalisée sous une pression comprise de préférence entre 100 et 300 bars et lors d'une étape ii) réalisée sous une pression comprise de préférence entre 10 et 60 bars,
c) application, à la base liquide obtenue à l'issue de l'étape a) ou à l'issue de l'étape b), d'un traitement thermique à une température comprise entre 60°C et 110°C pendant une durée suffisante pour pasteuriser ladite base,
d) séchage par atomisation du mélange obtenu à l'issue de l'étape c), et
e) récupération de la composition pasteurisée sous forme de poudre obtenue à l'issue de l'étape d).

L'étape a) de préparation d'une base liquide comprend le mélange, sous agitation, des composants, ingrédients ou éléments d'intérêt, tels que décrits précédemment, constitutifs de la composition.

La réalisation d'un mélange liquide passe par la dilution de chaque ingrédient dans de l'eau.

Les ingrédients (base de la composition, par exemple base de lait infantile ou base de composition diététique, et agent(s) épaississant(s)) peuvent être mélangés sous forme de poudres et mis en solution par la suite. Ils peuvent aussi être mélangés sous forme de solutions. Il est également envisageable d'ajouter l'un des composants sous forme de poudre à l'autre composant qui se trouve en solution. Il est dans ce cas préférable de maintenir le composant en solution sous agitation lors du mélange au composant sous forme de poudre afin de limiter ou réduire, idéalement d'éviter la formation d'agglomérats lors du mélange.

Ainsi, le caractère aqueux du mélange obtenu à l'issue de l'étape a) peut provenir de la forme liquide des agents épaississants, de la forme liquide de la base utilisée et/ou de l'adjonction d'eau au mélange des produits utilisés sous forme de poudre.

Un agitateur ou une unité de mélange, par exemple une pompe de mélange, un défloculateur, ou un mélangeur équipé d'un système rotor/stator, peut avantageusement être utilisé pour dissoudre les différents ingrédients et faciliter l'obtention d'une base homogène.

L'utilisation préférée d'un mélangeur de forme et taille adaptées permet également d'éviter l'incorporation excessive d'air dans la base liquide, en particulier la base de lait.

L'homme du métier sera par ailleurs en mesure d'adapter les vitesses de rotation pour diminuer encore davantage une telle incorporation excessive d'air dans la base liquide.

L'étape a) de mélange est de préférence réalisée à une température d'au moins 60°C, par exemple comprise entre environ 60°C et 90°C ou entre environ 60 °C et 80 °C, encore plus préférentiellement entre environ 70 °C et 75°C. Typiquement, la température est de 75°C.

Selon un aspect préféré, la base liquide obtenue à l'issue de l'étape a) est maintenue sous agitation jusqu'à l'application de l'étape d'homogénéisation, par exemple à l'aide d'un dispositif tel que décrit précédemment.

L'étape d'homogénéisation de la base liquide mélangée, éventuellement pasteurisée, obtenu à l'issue de l'étape a) du procédé décrit permet le fractionnement des éléments constitutifs de ladite base. L'homogénéisation comprend deux étapes de compression destinée à renforcer la stabilité de cette base. La première étape i) de fractionnement est de préférence réalisée sous une pression comprise entre environ 100 bars et environ 300 bars, la deuxième étape ii) étant de préférence réalisée sous une pression comprise entre 10 bars environ et 60 bars environ.

Cette étape est avantageusement mise en œuvre sur un homogénéisateur à deux étages.

La pression d'homogénéisation du premier étage dudit homogénéisateur est ainsi typiquement comprise entre environ 100 bars et environ 300 bars, de préférence entre environ 150 bars et environ 300 bars, encore plus préférentiellement entre environ 170 bars et environ 200 bars.

La pression d'homogénéisation du second étage est typiquement comprise entre environ 10 bars et environ 60 bars, de préférence entre environ 30 bars et environ 60 bars, encore plus préférentiellement entre environ 30 bars et environ 40 bars.

L'étape de pasteurisation, éventuellement présente dans le procédé de préparation décrit, prévoit l'application, au mélange obtenu à l'issu de l'étape a) ou à l'issue de l'étape d'homogénéisation, d'un traitement thermique compris entre environ 70°C et environ 110°C, de préférence entre 70°C et 100°C, entre 75°C et 100°C, entre 75°C et 95°C, entre 80°C et 95°C ou entre 85°C et 95°C, encore plus préférentiellement entre 80°C et 90°C, pendant une durée suffisante pour inactiver et détruire au moins les germes considérés comme pathogènes (notamment *C. Sakazakii).*

Typiquement, le traitement thermique de l'étape de pasteurisation est appliqué pendant au moins 2 minutes, et de préférence au plus 10 minutes, lorsque la température est égale ou inférieure à 80°C, par exemple comprise entre 75°C et 60°C, et pendant au moins 25 secondes, typiquement au moins 1 minute, et de préférence au plus 5 minutes, lorsque la température est égale ou supérieure à 85°C, par exemple comprise entre 85°C et 100°C.

Le traitement peut également être appliqué pendant une durée supérieure à 2 minutes, et de préférence inférieure à 10 minutes, lorsque la température est de 75°C, pendant une durée comprise entre environ 2 minutes et environ 3 minutes lorsque la température est de 80°C, pendant une durée comprise entre environ 1 minute et environ 2 minutes lorsque la température est de 90°C, pendant une durée d'environ 1 minute lorsque la température est de 95°C, et de moins de 30 secondes, typiquement de 25 secondes, lorsque la température est de 100°C.

Le mélange, pasteurisé ou non, obtenu à l'issue de l'étape d'homogénéisation du procédé décrit est avantageusement séché par atomisation afin d'obtenir une composition sous forme de poudre comprenant, comme expliqué précédemment, un extrait sec compris entre 85% et 99%, de préférence un extrait sec d'au moins 94% ou d'au moins 95%, encore plus préférentiellement d'au moins 98% par rapport au poids total de lait sous forme de poudre.

Le mélange, pasteurisé ou non, obtenu à l'issue de l'étape d'homogénéisation du procédé décrit est typiquement introduit au sommet d'une tour d'atomisation.

Le mélange est alors "atomisé" (transformé en aérosol ou brouillard) au moyen d'une turbine d'atomisation ou par injection à haute pression au travers d'une ou plusieurs buses. Avantageusement, l'atomisation est réalisée sous une pression comprise entre 120 et 230 bars, de préférence entre 135 et 195 bars. Les gouttelettes ainsi formées sont entraînées et déshydratées par un courant d'air chaud dont la température est typiquement comprise entre 160°C et 240°C, de préférence entre 180°C et 220°C. Les gouttelettes sont séchées en une poudre avant de tomber sur les parois inférieures de l'appareil. La séparation poudre - air humide est obtenue par exemple à l'aide de séparateurs cyclone dont l'utilisation est bien connue de l'homme de l'art.

Lorsque l'on souhaite obtenir une composition en poudre selon un tel procédé, la déshydratation dans la tour d'atomisation doit de préférence ne pas être totale. L'humidité résiduelle présente dans la composition en poudre peut par exemple être comprise entre 6 et 14% en bas de chambre. Cette humidité résiduelle permet une agglomération limitée et contrôlée des particules qui conduit à la formation de granulés à structure poreuse.

La déshydratation peut ensuite être achevée dans des dispositifs complémentaires de type sécheurs à lit fluidisé. La poudre peut ensuite être refroidie à l'intérieur d'un lit vibro-fluidisé.

L'invention revendiquée est illustrée de façon non limitative par les figures et exemples ci-dessous.

### LEGENDE DE LA FIGURE

**Figure 1** **:**
A - tableau de viscosité de la composition en fonction du pH
B - graphe de viscosité de la composition selon l'invention en fonction du pH

### EXEMPLES

### EXEMPLE 1 : Formulation

La formulation des laits destinés à l'alimentation infantile est le plus souvent strictement encadrée par une législation fixant des normes de composition. Selon les pays, il peut y avoir des différences d'appréciation en raison notamment de spécificités locales dans la pratique de la diversification alimentaire ou bien des modifications mineures des optimums nutritionnels résultants de travaux ou d'études conduites localement. Dans ces conditions, le présent exemple n'entend pas représenter la diversité des formulations de laits épaissies selon l'invention.

Classiquement, pour répondre aux besoins nutritionnels du nourrisson, les laits infantiles comprennent environ 10-15% de protéines, environ 25% de lipides et environ 50 à 65% de glucides ainsi que des minéraux, des vitamines et éventuellement des facteurs de croissance. D'autres ingrédients tels que par exemple un ou plusieurs prébiotiques et/ou probiotiques peuvent par ailleurs être ajoutés aux laits infantiles.

Un exemple non limitatif de composition de type lait infantile anti-régurgitation et/ou anti-reflux préservant le transit intestinal selon l'invention est fourni dans le tableau I ci-dessous.

**Tableau I :**

| **Composition / Profil Nutritionnel:** | | **Pour 100 g de poudre** | **Pour 100 ml de lait reconstitué à 13%** |
|---|---|---|---|
| **Protéines Nx 6,25** | g | 12,1 | 1,57 |
| **Lipides** | g | 25,1 | 3,26 |
| **Glucides** | g | 53 | 6,89 |
| *Maltodextrines* | g | 26 | 3,38 |
| *Lactose* | g | 22 | 2,86 |
| *Amidon* | g | 0,9 | 0,12 |
| *Dextrose* | g | 3,4 | 0,44 |
| ***Pectine HM*** | g | 1 | 0,13 |
| ***Pectine LM amidée*** | g | 4,25 | 0,55 |
| ***Caroube soluble à froid*** | g | 0,5 | 0,07 |
| **Energie** | kcal | 493,7 | 64,18 |

| **Minéraux** | | | |
|---|---|---|---|
| Sodium | mg | 180 | 23,40 |
| Potassium | mg | 520 | 67,60 |
| Chlore | mg | 345 | 44,85 |
| Calcium | mg | 460 | 59,80 |
| Phosphore | mg | 340 | 44,20 |
| Magnesium | mg | 45 | 5,85 |
| Fer | mg | 6 | 0,78 |
| Zinc | mg | 3,5 | 0,46 |
| Iode | µg | 55 | 7,15 |
| Cuivre | µg | 350 | 45,50 |
| Manganese | µg | 45 | 5,85 |
| Selenium | µg | 9 | 1,17 |
| chrome | µg | <45 | <5,85 |
| molybdene | µg | <45 | <5,85 |
| fluor | µg | <450 | <58,5 |

| **Vitamines** | | | |
|---|---|---|---|
| A | µg RE | 450 | 58,50 |
| B1 | µg | 400 | 52,00 |
| B2 | µg | 800 | 104,00 |
| B6 | µg | 300 | 39,00 |
| B12 | µg | 1,5 | 0,20 |
| C | mg | 60 | 7,80 |
| D | µg | 7,5 | 0,98 |
| E | IU | 16,4 | 2,13 |
| K1 | µg | 30 | 3,90 |
| Biotine | µg | 15 | 1,95 |
| Niacine | mg | 4,5 | 0,59 |
| acide folique | µg | 60 | 7,80 |
| acide pantothénique | mg | 2,4 | 0,31 |
| Choline | mg | 60 | 7,80 |
| Inositol | mg | 30 | 3,90 |
| Taurine | mg | 44 | 5,72 |
| L-carnitine | mg | 8 | 1,04 |

Dans la présente composition, l'amidon, en particulier l'amidon précuit et/ou pré-gélatinisé, présent à faible taux, n'a pas d'influence sur l'efficacité de la formule, en particulier sur sa viscosité.

En effet, lors de la fabrication des compositions selon l'invention, l'amidon, en particulier l'amidon précuit et/ou pré-gélatinisé, lorsqu'il est présent, subit, comme l'ensemble des ingrédients constituant la composition, en particulier la base liquide, une étape de chauffage à une température d'au moins 60°C, suivie d'une homogénéisation par fractionnement des éléments constitutifs de la composition, en particulier de la base liquide, lors d'une première étape i) réalisée sous une pression comprise entre 100 et 300 bars et lors d'une étape ii) réalisée sous une pression comprise entre 10 et 60 bars. De tels traitements inhibent ou suppriment toute capacité de l'amidon à épaissir et à impacter la viscosité des compositions selon l'invention qui comprendraient de l'amidon.

### EXEMPLE 2 : Procédé de fabrication d'un lait infantile pasteurisé sous forme de poudre comprenant i) de la caroube soluble à froid et ii) au moins deux pectines de natures différentes :

La base de lait infantile comprenant 37% d'extrait sec est préparée en mélangeant de l'eau préalablement chauffée à 70°C avec les différents ingrédients du lait infantile (lesdits ingrédients comprenant protéines ou acides aminés + caroube soluble à froid + pectines faiblement estérifiées ou pectines faiblement estérifiées et amidées + pectines fortement estérifiées + glucides + minéraux + matières grasses végétales + vitamines + facteurs de croissance). La caroube et les pectines sont incorporées dans la base de lait infantile maintenue sous agitation afin d'obtenir leur dissolution complète. L'ensemble est maintenu à 70°C sous agitation dans une cuve à double paroi jusqu'à l'étape d'homogénéisation. La base de lait infantile subit ensuite une homogénéisation double effet à 200/40 bars, i.e., une première étape d'homogénéisation est réalisée sous une pression de 200 bars et la deuxième étape d'homogénéisation est réalisée sous une pression de 40 bars. La base de lait infantile homogénéisée est ensuite de préférence pasteurisée par traitement thermique à environ 80°C pendant 1 à 2 minutes dans le but d'éliminer les risques bactériologiques, en particulier ceux liés à *Cronobacter Sakazakii.*

La base de lait infantile, de préférence pasteurisée, subie ensuite une étape d'atomisation réalisée sous une pression de 190 bars qui permet d'obtenir des gouttelettes d'un diamètre suffisamment faible pour être séchée avec de l'air dont la température à l'entrée dans la chambre est de 185°C et la température à la sortie de la chambre est de 94°C.

Le procédé mis en œuvre permet ici d'obtenir un débit de 1000 et 2000 kg de poudre/heure.

Le lait liquide reconstitué (prêt à la consommation) obtenu à partir de cette poudre de lait infantile a un extrait sec d'environ 13% dans le biberon. La viscosité de ce lait reconstitué, mesurée à 60 rpm (rotations par minute), à 37°C, est comprise entre 25-45 mPa.s (mobile S61) à un pH proche de la neutralité et comprise entre 1502 et 145 mPa.s pour une gamme de pH allant de 6 à 3,5. Le lait liquide reconstitué contient 0,68% de pectines et 0.065% de caroube dans le biberon.

### EXEMPLE 3 : Comparaison entre la viscosité d'un lait reconstitué comprenant un lait sous forme de poudre selon l'invention à la viscosité d'un lait AR classique contenant de l'amidon (Novalac AR®) :

Un lait infantile selon l'invention est réalisé et recueilli sous forme de poudre selon le procédé de fabrication mentionné dans l'exemple 2. Ce lait selon l'invention comprend ainsi 4,25% de pectines faiblement estérifiées ou de pectines faiblement estérifiées et amidées, 1% de pectines hautement estérifiées et 0.5% de caroube soluble à froid.

### Méthode et outils:

Le lait infantile liquide est alors préparé, par dilution du lait infantile sous forme de poudre selon l'invention dans de l'eau chaude (à 60°C) à 13 % d'extrait sec. Ledit lait infantile liquide est ensuite refroidi à 37°C.
Le lait AR classique à base d'amidon est aussi préparé par dilution de la poudre dans de l'eau (également à 37°C) à 13% d'extrait sec.
Les viscosités des deux produits sont ensuite mesurées à l'aide d'un viscosimètre Brookfield (DV-I Prime) au pH de reconstitution (proche de la neutralité) avec un mobile S61 à 60 rpm (rotations par minute) et à une température de 37°C.

De l'acide chlorhydrique, de molarité égale à 1 (HCl 1M), est ajouté aux deux produits reconstitués pour atteindre un pH de 5,5. Les viscosités des deux produits acidifiés sont alors mesurées à 37°C, avec un mobile S61 et à une vitesse de 60 rpm pour le produit Novalac AR® et avec un mobile S62 et à une vitesse de 30 rpm pour la formule de lait infantile selon l'invention.
Puis de l'acide chlorhydrique, de molarité égale à 1 (HCl 1M), est de nouveau ajouté aux deux produits reconstitués pour atteindre un pH de 3,5. Les viscosités des deux produits acidifiés sont alors de nouveau mesurées à 37°C, avec un mobile S62 et à une vitesse de 60 rpm.

**Tableau 3 :**

| | **Viscosités en** mPa.s | | |
|---|---|---|---|
| **Produits AR** | ***pH neutre*** | ***pH*=*5,5*** | ***pH*=*3,5*** |
| Novalac AR® | 10 | 96 | 241 |
| Lait infantile selon l'invention | 36 | 645 | 145 |

Conclusion : L'augmentation importante de viscosité, observée dès pH=6, en particulier à pH=5,5, du lait infantile selon l'invention permettra une efficacité importante contre les régurgitations et/ou reflux du nourrisson ou de l'enfant. En effet, le pH de l'estomac du nourrisson 15 min après l'ingestion d'un lait se trouve en général entre 6 et 5. On voit donc bien que le lait Novalac AR® classique, dont la viscosité est d'environ 100 mPa.s, aura un effet moindre sur les régurgitations et/ou reflux : plus le produit sera visqueux, plus les régurgitations et/ou reflux diminueront.

### EXEMPLE 4 : Comparaison entre la viscosité d'un lait reconstitué comprenant un lait sous forme de poudre selon l'invention à la viscosité d'un lait AR contenant de l'amidon et de la caroube soluble à froid (Novalac AR Digest®) :

Un lait infantile selon l'invention est réalisé et recueilli sous forme de poudre selon le procédé de fabrication mentionné dans l'exemple 2. Ce lait selon l'invention contient ainsi 4,25% de pectines faiblement estérifiées ou de pectines faiblement estérifiées et amidées, 1% de pectines hautement estérifiées et 0.5% de caroube soluble à froid.

### Méthode et outils:

Le lait infantile liquide selon l'invention est alors réalisé, par dilution du lait infantile sous forme de poudre selon l'invention dans de l'eau chaude (à 60°C) à 13 % d'extrait sec. Ledit lait infantile liquide est ensuite refroidi à 37°C.

Le lait AR-Digest® à base d'amidon et de caroube soluble à froid est aussi préparé par dilution de la poudre dans de l'eau (également à 37°C) à 13% d'extrait sec.

Les viscosités des deux produits sont ensuite mesurées à l'aide d'un viscosimètre Brookfield (DV-I Prime) au pH de reconstitution (proche de la neutralité) avec un mobile S61 à 60 rpm (rotations par minute) et à une température de 37°C.

De l'acide chlorhydrique, de molarité égale à 1 (HCl 1M), est ajouté aux deux produits reconstitués pour atteindre un pH de 5,5. Les viscosités des deux produits acidifiés sont alors mesurées à 37°C, avec un mobile S62, et à une vitesse de 60 rpm pour le produit Novalac AR-Digest® et à une vitesse de 30 rpm pour la formule de lait infantile selon l'invention.

Puis de l'acide chlorhydrique, de molarité égale à 1 (HCl 1M), est de nouveau ajouté aux deux produits reconstitués pour atteindre un pH de 3,5. Les viscosités des deux produits acidifiés sont alors de nouveau mesurées à 37°C, avec un mobile S62 et à une vitesse de 60 rpm.

**Tableau 4 :**

| | **Viscosités en mPa.s** | | |
|---|---|---|---|
| **Produits AR** | ***pH neutre*** | ***pH*=*5,5*** | ***pH*=*3,5*** |
| Novalac AR Digest® (protéines hydrolysées+ caroube soluble à froid (4%)) | 18 | 108 | 210 |
| Lait infantile selon l'invention | 36 | 645 | 145 |

Conclusion : Au même titre que dans l'exemple 3, on voit bien ici que le lait infantile selon l'invention permet une efficacité supérieure quant à la diminution des régurgitations et/ou reflux du nourrisson ou de l'enfant à des pH compris entre 6 et 5 (pH de l'estomac du nourrisson, environ 15 minutes après ingestion d'un lait infantile).

### EXEMPLE 5 : Comparaison entre la viscosité d'un lait reconstitué comprenant un lait sous forme de poudre selon l'invention obtenu par mélange à sec à la viscosité d'un lait AR classique épaissi à l'aide d'amidon et de caroube soluble à froid (Novalac AR Digest®) :

Une base de lait infantile est réalisée et recueillie sous forme de poudre en sortie de tour de séchage. A cette base se présentant sous forme de poudre sont ensuite ajoutés par mélange à sec, 4.25% de pectines faiblement estérifiées ou de pectines faiblement estérifiées et amidées, 1% de pectines fortement estérifiées et 0,5% de caroube soluble à froid.

### Méthode et outils:

Le lait infantile liquide est alors réalisé, par dilution du lait infantile sous forme de poudre selon l'invention dans de l'eau chaude (à 60°C) à 13 % d'extrait sec. Ledit lait infantile liquide est ensuite refroidi à 37°C.
Le lait AR-Digest® à base d'amidon et de caroube soluble à froid est aussi préparé par dilution de la poudre dans de l'eau (également à 37°C) à 13% d'extrait sec.

Les viscosités des deux produits sont ensuite mesurées à l'aide d'un viscosimètre Brookfield (DV-I Prime) au pH de reconstitution (proche de la neutralité) avec un mobile S61 à 60 rpm (rotations par minute) et à une température de 37°C.
De l'acide chlorhydrique, de molarité égale à 1 (HCl 1M), est ajouté aux deux produits reconstitués pour atteindre un pH de 5,5. Les viscosités des deux produits acidifiés sont alors mesurées à 37°C, avec un mobile S62, et à une vitesse de 60 rpm pour le produit Novalac AR-Digest® et à une vitesse de 30 rpm pour la formule de lait infantile selon l'invention.
Puis de l'acide chlorhydrique, de molarité égale à 1 (HCl 1M), est de nouveau ajouté aux deux produits reconstitués pour atteindre un pH de 3,5. Les viscosités des deux produits acidifiés sont alors de nouveau mesurées à 37°C, avec un mobile S62 et à une vitesse de 60 rpm.

**Tableau 5 :**

| | **Viscosités en mPa.s** | | |
|---|---|---|---|
| **Produits AR** | ***pH neutre*** | ***pH*=*5,5*** | ***pH*=*3,5*** |
| Novalac AR Digest® (caroube soluble à froid (4%) + amidon) | 18 | 108 | 210 |
| Lait infantile selon l'invention obtenu par mélange à sec | 25 | 530 | 125 |

### Conclusion :

Comme dans l'exemple précédent, le lait infantile selon l'invention permet une efficacité supérieure quant à la diminution des régurgitations et/ou reflux du nourrisson ou de l'enfant à des pH compris entre 6 et 5 (pH de l'estomac du nourrisson, 15 minutes après ingestion d'un lait infantile).

### EXEMPLE 6 : Evaluation de l'efficacité clinique sur les régurgitations et de l'effet sur le transit intestinal d'un lait reconstitué obtenu à partir d'une composition sous forme de poudre selon l'invention

Une étude clinique ouverte a été réalisée afin de déterminer l'influence d'un lait reconstitué obtenu à partir d'une composition, typiquement d'un lait, sous forme de poudre selon l'invention, sur la fréquence et sur l'intensité des épisodes de régurgitations et/ou de reflux ainsi que sur le transit intestinal, en particulier sur la consistance des selles des nourrissons. Cette étude clinique a été réalisée sur une durée de 14 jours plus ou moins 2 jours, c'est-à-dire sur une durée allant de 12 à 16 jours entre le jour de la première visite ou jour de l'inclusion, noté J0, et le jour de la deuxième visite, environ 14 jours plus tard, à plus ou moins deux jours, noté J14.

### Méthode et outils:

Une composition selon l'invention dont le profil nutritionnel correspond à celui du Tableau II ci-dessous a été préparée et recueillie sous forme de poudre.

**Tableau II :**

| **Composition / Profil Nutritionnel:** | | **Pour 100 g de poudre** | **Pour 100 ml de lait reconstitué à 13%** |
|---|---|---|---|
| **Protéines Nx 6,25** | g | 12,1 | 1,57 |
| **Lipides** | g | 25,1 | 3,26 |
| **Glucides** | g | 53 | 6,89 |
| ***Pectine HM*** | g | 1 | 0,13 |
| ***Pectine LM amidée*** | g | 4,25 | 0,55 |
| ***Caroube soluble à froid*** | g | 0,5 | 0,07 |
| **Energie** | kcal | 493,7 | 64,18 |

| **Minéraux** | | | |
|---|---|---|---|
| Sodium | mg | 180 | 23,40 |
| Potassium | mg | 520 | 67,60 |
| Chlore | mg | 345 | 44,85 |
| Calcium | mg | 460 | 59,80 |
| Phosphore | mg | 340 | 44,20 |
| Magnesium | mg | 45 | 5,85 |
| Fer | mg | 6 | 0,78 |
| Zinc | mg | 3,5 | 0,46 |
| Iode | µg | 55 | 7,15 |
| Cuivre | µg | 350 | 45,50 |
| Manganese | µg | 45 | 5,85 |
| Selenium | µg | 9 | 1,17 |
| chrome | µg | <45 | <5,85 |
| molybdene | µg | <45 | <5,85 |
| fluor | µg | <450 | <58,5 |

| **Vitamines** | | | |
|---|---|---|---|
| A | µg RE | 450 | 58,50 |
| B1 | µg | 400 | 52,00 |
| B2 | µg | 800 | 104,00 |
| B6 | µg | 300 | 39,00 |
| B12 | µg | 1,5 | 0,20 |
| C | mg | 60 | 7,80 |
| D | µg | 7,5 | 0,98 |
| E | IU | 16,4 | 2,13 |
| K1 | µg | 30 | 3,90 |
| Biotine | µg | 15 | 1,95 |
| Niacine | mg | 4,5 | 0,59 |
| acide folique | µg | 60 | 7,80 |
| acide pantothénique | mg | 2,4 | 0,31 |
| Choline | mg | 60 | 7,80 |
| Inositol | mg | 30 | 3,90 |
| Taurine | mg | 44 | 5,72 |
| L-carnitine | mg | 8 | 1,04 |

Pour cela, une base de lait infantile comprenant 37% d'extrait sec est préparée en mélangeant de l'eau préalablement chauffée à 70°C avec les différents ingrédients du lait infantile (lesdits ingrédients comprenant protéines ou acides aminés + caroube soluble à froid + pectines faiblement estérifiées ou pectines faiblement estérifiées et amidées + pectines fortement estérifiées + glucides + minéraux + matières grasses végétales + vitamines + facteurs de croissance). La caroube et les pectines sont incorporées dans la base de lait infantile maintenue sous agitation afin d'obtenir leur dissolution complète. L'ensemble est maintenu à 70°C sous agitation dans une cuve à double paroi jusqu'à l'étape d'homogénéisation. La base de lait infantile est ensuite pasteurisée par traitement thermique à environ 80°C pendant 1 à 2 minutes dans le but d'éliminer les risques bactériologiques, en particulier ceux liés à *Cronobacter Sakazakii.* La base de lait infantile pasteurisée subit ensuite une homogénéisation double effet à 200/40 bars, i.e., une première étape d'homogénéisation est réalisée sous une pression de 200 bars et la deuxième étape d'homogénéisation est réalisée sous une pression de 40 bars.
La base de lait infantile pasteurisée et homogénéisée, subit ensuite de préférence une étape d'atomisation réalisée sous une pression de 140 bars qui permet d'obtenir des gouttelettes d'un diamètre suffisamment faible pour être séchée avec de l'air dont la température à l'entrée dans la chambre est de 185°C et la température à la sortie de la chambre est comprise entre 80 et 105°C, en particulier égale à 94°C.
Le procédé mis en œuvre permet ici d'obtenir un débit de 1000 et 2000 kg de poudre/heure.
La composition sous forme de poudre obtenue est ensuite conditionnée dans des boîtes. Cette composition selon l'invention comprend ainsi 4,25% de pectines faiblement estérifiées et amidées, 1% de pectines hautement estérifiées et 0,5% de caroube soluble à froid.
La concentration en protéines non hydrolysées est avantageusement d'environ 9% en poids (de préférence 7,2% environ de caséines entières et 1,8% de protéines solubles entières) et la concentration de protéines hydrolysées est avantageusement d'environ 3% en poids (de préférence exclusivement des protéines solubles), par rapport au poids total de la composition.

Pour être jugés éligibles pour cette étude, les nourrissons devaient être âgés au maximum de 5 mois, être nourris exclusivement par formule infantile, présenter au moins 5 épisodes de régurgitation par jour, ne pas avoir débuté la diversification alimentaire et ne pas introduire un nouvel aliment dans leur alimentation dans les 2 semaines suivant l'inclusion.
Pour être jugés éligibles pour cette étude les nourrissons devaient également ne reproduire aucun des critères de non-inclusion suivants :
- être alimentés au lait maternel,
- présenter des symptômes de Reflux Gastro-Œsophagien (RGO) compliqués tels que nausées, dysphagie, retard de croissance, signes évidents d'œsophagite,
- présenter des troubles intestinaux tels que notamment gastroentérite ou diarrhées chroniques,
- avoir reçu un traitement médical pour les régurgitations au cours de la semaine précédent l'inclusion (tel que Omeprazole ou Ranitidine), ou devoir débuter un tel traitement,
- avoir reçu des antibiotiques au cours de la semaine précédent l'inclusion ou nécessiter une antibiothérapie,
- présenter une allergie aux protéines de lait de vache, ou présenter un risque accru d'allergie aux protéines de lait de vache,
- présenter une situation qui selon l'investigateur pourrait interférer avec la conduite de l'étude ou présenter un risque particulier pour eux, ou
- participer à une autre étude clinique.

100 nourrissons éligibles ont été recrutés. Sur ces 100 nourrissons recrutés initialement, 10 nourrissons ont été exclus avant J14 pour des raisons telles que changement d'avis des parents, suspicion d'allergie aux protéines de lait de vache, reprise de l'allaitement, perte de vue du patient, formule refusée *a priori* en raison de son goût, suspicion de RGO compliqué, suspicion de gastro-entérite. Les résultats présentés sont ceux associés aux 90 nourrissons inclus avant J14, qui représentent 100% des cas évalués.
Le critère principal étudié lors de cette étude clinique était la fréquence des régurgitations, en particulier le nombre de régurgitations par jour. Les régurgitations ont également été évaluées à l'aide du score de Vandenplas, défini de la façon suivante :
Score = 0 : moins de 2 épisodes de régurgitations par jour
Score = 1 : au moins 3 et au plus 5 épisodes de régurgitations d'un petit volume par jour (moins d'une cuillère à café par jour, soit moins de 5 ml environ)
Score = 2 : plus de 5 épisodes de régurgitations par jour dont le volume est supérieur à celui d'une cuillère à café
Score = 3 : plus de 5 épisodes de régurgitations par jour d'environ la moitié du volume du biberon consommé pour moins de la moitié des biberons consommés.
Score = 4 : Régurgitations continues de petits volumes durant plus de 30 minutes après chaque biberon
Score = 5 : Régurgitations d'au moins la moitié du volume du biberon consommé pour au moins la moitié des biberons consommés
Score = 6 : Régurgitation du volume total du biberon consommé après chaque biberon
Le score de Vandenplas a été utilisé dans plusieurs études cliniques à ce jour et est décrit dans plusieurs publications scientifiques, notamment dans la publication *«* Vandenplas Y, Hachimi-Idrissi S, Casteels A, Mahler T, Loeb H. A clinical trial with an "anti-regurgitation"formula. Eur J Pediatr. 1994; 153(6): 419-23 *».*
L'homme du métier connaît d'autres scores évaluant l'intensité des régurgitations, en particulier le volume et/ou la fréquence des régurgitations, tel que par exemple le score d'Orenstein décrit dans la publication « Orenstein SR, Magill HL, Brooks P. Thickening of infant feedings for therapy of gastroesophageal reflux. J Pediatr 1987;110:181-6 *».*

L'effet sur le transit a été mesuré par l'évaluation de la consistance moyenne des selles des nourrissons, à choisir parmi selles dures, selles formées, selles molles ou liquides.
Lors d'une première visite médicale au jour J0, les parents des 100 nourrissons ont signé un consentement éclairé, chacun des 100 nourrissons inclus dans l'étude a été examiné par un investigateur pédiatre qui a rempli un cahier d'observations dans lequel ont été reportées différentes données, en particulier, le nombre moyen de régurgitations par jour, la consistance moyenne des selles et le score de Vandenplas moyen, en moyenne sur les 3 jours précédents la visite médicale.
Lors de cette première visite, le pédiatre a remis aux parents des boîtes de lait reconstitué comprenant un lait sous forme de poudre selon l'invention en quantité suffisante pour couvrir les besoins du nourrisson pendant 2 semaines. Le lait reconstitué comprenant un lait sous forme de poudre selon l'invention contenu dans ces boîtes a été la seule alimentation des nourrissons pendant toute la durée de l'étude.
Durant toute la durée de l'étude et pour chaque préparation de biberon, les parents avaient pour instruction de préparer le lait infantile liquide, par dilution du lait infantile sous forme de poudre selon l'invention, dans de l'eau à 40°C à 13 % d'extrait sec et de le laisser refroidir ensuite à environ 37°C. Ils avaient également pour instructions de ne pas changer les habitudes alimentaires du nourrisson et de continuer à le nourrir comme d'habitude. Typiquement, ils avaient pour instructions de ne pas changer le nombre ou le volume des biberons et de respecter les demandes alimentaires du nourrisson.

A J14, soit environ 14 jours après inclusion, à plus ou moins 2 jours, une deuxième visite médicale a eu lieu pour les 90 nourrissons inclus avant J14. Lors de cette visite, l'investigateur pédiatre a complété le cahier d'observations dans lequel ont été reportées différentes données, en particulier, le nombre moyen de régurgitations par jour, la consistance moyenne des selles et le score de Vandenplas moyen, en moyenne sur les 3 jours précédents la visite médicale.

### Fréquence des régurgitations

Les informations du cahier d'observations de l'investigateur pédiatre concernant le nombre moyen de régurgitations par jour à J0 et à J14 pour les 90 nourrissons inclus avant J14 ont été répertoriées dans le tableau 6 ci-dessous.

**Tableau 6 :**

| | **Nombre de régurgitations par jour** | |
|---|---|---|
| | *Inclusion* = *J0* | *J14* |
| Moyenne | 7.3 | 1.06 |
| Ecart-type | 3.4 | 1.25 |
| Minimum | 5 | 0 |
| Maximum | 20 | 5 |

Il a également été observé une diminution du nombre de régurgitations entre J0 et J14 dans 100% des cas évalués. Alors que 100% des nourrissons évalués avaient au moins 5 épisodes de régurgitations par jour à J0, 70% de ces nourrissons avaient moins de 0 ou 1 épisode de régurgitation par jour à J14.

### Conclusion :

Ces données confirment l'efficacité d'un lait reconstitué comprenant une composition sous forme de poudre selon l'invention sur la diminution du nombre de régurgitations et/ou reflux du nourrisson par jour.

### Intensité des régurgitations

Les informations du cahier d'observations de l'investigateur pédiatre concernant le score de Vandenplas moyen à J0 et à J14 pour les 90 nourrissons inclus avant J14 indiquent une diminution du score de Vandenplas dans 99% des cas.

Il a également été observé une diminution de la valeur moyenne du score de Vandenplas entre J0 et J14 qui passe de 1,9 à 0,2, comme indiqué dans le tableau 7 ci-dessous.

A J14, 77 nourrissons, soit 85,5% des 90 nourrissons inclus, ont un score de Vandenplas de 0 et ont donc moins de 2 épisodes de régurgitation par jour.

**Tableau 7 :**

| | **Score de Vandenplas** | |
|---|---|---|
| | *Inclusion* = *J0* | *J14* |
| Moyenne | 1.9 | 0.2 |
| Ecart-type | 0.8 | 0.6 |

### Conclusion :

Ces données confirment l'efficacité d'un lait reconstitué comprenant un lait sous forme de poudre selon l'invention sur la diminution de l'intensité des régurgitations et/ou reflux du nourrisson.

### Transit intestinal

Les informations du cahier d'observations de l'investigateur pédiatre concernant la consistance moyenne des selles à J0 et à J14 pour les 90 nourrissons inclus avant J14 ont été répertoriées dans le tableau 8 ci-dessous.

**Tableau 8 :**

| | **Consistance des selles** | |
|---|---|---|
| | *Inclusion* | *J14* |
| Dures | 14.40% | 3.40% |
| Formées | 36.70% | 51.10% |
| Molles à liquides | 48.90% | 45.50% |

### Conclusion :

Ces données confirment qu'un lait reconstitué comprenant un lait sous forme de poudre selon l'invention n'altère pas le transit intestinal du nourrisson, typiquement n'entraîne pas d'effets indésirables sur les selles, tels que l'augmentation du pourcentage de constipation, diarrhée, selles dures et/ou molles et/ou liquides et/ou trop fréquentes observé au sein de la population d'enfants testés.
Ces données montrent également une amélioration voire une normalisation de la consistance des selles entre J0 et J14 comme illustré par une diminution des pourcentages de nourrissons ayant des selles dures, molles ou liquides et une augmentation du pourcentage de nourrissons ayant des selles formées. Le lait infantile selon l'invention permet donc la restauration du transit intestinal chez des nourrissons c'est-à-dire qu'il est capable pour ces patients de rétablir un transit normal, de préférence des selles formées, lorsque celui-ci est altéré.
La composition sous forme de poudre selon l'invention n'altère pas, préserve, voire restaure le transit intestinal du sujet auquel elle est administrée.

Le lait infantile selon l'invention est donc efficace comme lait anti-régurgitation et/ou anti-reflux, tant en ce qui concerne la fréquence des régurgitations et/ou du reflux que en ce qui concerne leur intensité et cela sans altérer le transit intestinal, typiquement en évitant aux nourrissons les désagréments associés à une altération du transit tels que constipations, selles molles, selles liquides et/ou trop fréquentes (diarrhées), et/ou coliques, voire même en restaurant le transit intestinal desdits nourrissons.

## Revendications

1. Composition nutritionnelle pour nourrisson ou enfant, **caractérisée en ce que** ladite composition comprend de la caroube soluble à froid présentant une solubilité en milieu aqueux supérieure à 60% à une température comprise entre 10°C et 45°C dont la concentration est comprise entre 0.2 et 0.75% en poids et au moins deux pectines de natures différentes dont 0.1 à 5% en poids de pectines hautement estérifiées, et 1 à 8 % en poids de pectines faiblement estérifiées, lesdits pourcentages étant exprimés en poids par rapport au poids total de composition.

2. Composition selon la revendication 1 **caractérisée en ce que** les pectines sont des pectines hautement méthylées dont le degré de méthylation est compris entre 50% et 90%, des pectines faiblement méthylées, en particulier des pectines amidées et faiblement méthylées dont le degré de méthylation est compris entre 20% et 50%, ou un mélange des deux.

3. Composition selon l'une quelconque des revendications précédentes, **caractérisée en ce qu'**elle comprend en outre de l'amidon.

4. Composition selon l'une des quelconque revendications précédentes, **caractérisée en ce qu'**elle comprend en outre des glucides, lesdits glucides étant de préférence du lactose et/ou des maltodextrines ; des lipides ; des protéines, lesdites protéines étant hydrolysées ou non ; et/ou des acides aminés.

5. Composition selon l'une quelconque des revendications précédentes, **caractérisée en ce qu'**elle se présente sous forme de poudre.

6. Composition selon l'une quelconque des revendications précédentes, **caractérisée en ce que** la composition est un lait infantile, une supplémentation alimentaire pour nourrisson, un lait de suite, un lait de croissance, ou un aliment diététique.

7. Méthode de préparation d'une composition nutritionnelle pour nourrisson ou enfant sous forme de poudre, ladite méthode comprenant les étapes de :
a) préparation d'une base liquide de composition dont la teneur en matière sèche est d'au moins 20% en poids par rapport au poids total de ladite composition, par mélange, sous agitation, à une température d'au moins 60°C, des éléments constitutifs de ladite composition, lesdits éléments comprenant au moins deux pectines de nature différentes dont 0.1 à 5% en poids de pectines hautement estérifiées et 1 à 8 % en poids de pectines faiblement estérifiées, et de la caroube soluble à froid présentant une solubilité en milieu aqueux supérieure à 60% à une température comprise entre 10°C et 45°C, la concentration en caroube soluble à froid étant comprise entre 0.2 et 0.75% en poids, lesdits pourcentages étant exprimés par rapport au poids total de ladite composition,
b) homogénéisation de la base liquide obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) séchage par atomisation du mélange obtenu à l'issue de l'étape b), et
d) récupération de la composition obtenue à l'issue de l'étape c) sous forme de poudre.

8. Méthode de préparation d'une composition nutritionnelle pour nourrisson ou enfant sous forme de poudre selon la revendication 7, **caractérisée en ce que** les au moins deux pectines de natures différentes correspondent à au moins une pectine faiblement méthylée, en particulier une pectine amidée et faiblement méthylée, dont le degré de méthylation est compris entre 20% et 50% et à au moins une pectine hautement méthylée dont le degré de méthylation est compris entre 50% et 90%.

9. Méthode de préparation d'une composition nutritionnelle pour nourrisson ou enfant sous forme de poudre selon la revendication 7 ou 8, comprenant en outre une étape d'application, à la base liquide obtenue à l'issue de l'étape a) ou à l'issue de l'étape b), d'un traitement thermique à une température comprise entre 60°C et 110°C pendant une durée suffisante pour pasteuriser ladite base, et permettant la récupération d'une composition pasteurisée sous forme de poudre à l'issue de l'étape d).

10. Méthode selon l'une quelconque des revendications 7 à 9, **caractérisée en ce que** la teneur en matière sèche de la base liquide est d'au moins 35% en poids par rapport au poids total de la base liquide.

11. Méthode selon l'une quelconque des revendications 7 à 10, **caractérisée en ce que** les éléments constitutifs de la composition comprennent en outre de l'amidon précuit et/ou prégélatinisé.

12. Composition nutritionnelle pour nourrisson ou enfant sous forme de poudre, **caractérisée en ce qu'**elle est susceptible d'être obtenue par une méthode selon l'une quelconque des revendications 7 à 11.

13. Composition selon la revendication 1 ou 12 pour son utilisation dans la prévention ou le traitement des régurgitations et/ou reflux du nourrisson ou de l'enfant.

14. Composition pour son utilisation selon la revendication 13 dans la prévention ou le traitement en outre des troubles intestinaux du nourrisson ou de l'enfant.

## Patentansprüche

1. Nährstoffzusammensetzung für Säuglinge oder Kinder, **dadurch gekennzeichnet, dass** besagte Zusammensetzung kalt lösliches Johannisbrot, das eine Löslichkeit in wässrigem Medium von größer als 60% bei einer Temperatur zwischen 10°C und 45°C aufweist, dessen Konzentration zwischen 0,2 und 0,75 Gew.-% beträgt, und wenigstens zwei verschiedenartige Pektine umfasst, darunter 0,1 bis 5 Gew.-% hoch veresterter Pektine und 1 bis 8 Gew.-% niedrig veresterter Pektine, wobei besagte prozentuale Anteile als Gewichtsprozente bezogen auf das Gesamtgewicht der Zusammensetzung ausgedrückt sind.

2. Zusammensetzung gemäß Anspruch 1, **dadurch gekennzeichnet, dass** die Pektine hoch methylierte Pektine, deren Methylierungsgrad zwischen 50% und 90% beträgt, niedrig methylierte Pektine, insbesondere amidierte und niedrig methylierte Pektine, deren Methylierungsgrad zwischen 20% und 50% beträgt, oder ein Gemisch davon sind.

3. Zusammensetzung gemäß einem der vorhergehenden Ansprüche, **dadurch gekennzeichnet, dass** sie außerdem Stärke umfasst.

4. Zusammensetzung gemäß einem der vorhergehenden Ansprüche, **dadurch gekennzeichnet, dass** sie außerdem Kohlenhydrate, wobei besagte Kohlenhydrate vorzugsweise Lactose und/oder Maltodextrine sind; Lipide; Proteine, wobei besagte Proteine hydrolysiert sein können; und/oder Aminosäuren umfasst.

5. Zusammensetzung gemäß einem der vorhergehenden Ansprüche, **dadurch gekennzeichnet, dass** sie in Pulverform vorliegt.

6. Zusammensetzung gemäß einem der vorhergehenden Ansprüche, **dadurch gekennzeichnet, dass** die Zusammensetzung eine Säuglingsmilch, eine Nahrungsergänzung für Säuglinge, eine Folgemilch, eine Wachstumsmilch oder ein diätetisches Nahrungsmittel ist.

7. Verfahren zur Herstellung einer Nährstoffzusammensetzung für Säuglinge oder Kinder in Pulverform, wobei besagtes Verfahren die Schritte umfasst:
a) Herstellung einer flüssigen Basis der Zusammensetzung, deren Trockenmassegehalt wenigstens 20 Gew.-% bezogen auf das Gesamtgewicht besagter Zusammensetzung beträgt, durch Mischen der konstitutiven Bestandteile besagter Zusammensetzung unter Rühren bei einer Temperatur von wenigstens 60°C, wobei besagte Bestandteile wenigstens zwei verschiedenartige Pektine, darunter 0,1 bis 5 Gew.-% hoch veresterter Pektine und 1 bis 8 Gew.-% niedrig veresterter Pektine, und kalt lösliches Johannisbrot umfassen, das eine Löslichkeit in wässrigem Medium von größer als 60% bei einer Temperatur zwischen 10°C und 45°C aufweist, wobei die Konzentration von kalt löslichem Johannisbrot zwischen 0,2 und 0,75 Gew.-% beträgt, wobei besagte prozentuale Anteile als Gewichtsprozente bezogen auf das Gesamtgewicht besagter Zusammensetzung ausgedrückt sind,
b) Homogenisation der aus Schritt a) erhaltenen flüssigen Basis durch Fraktionierung der konstitutiven Bestandteile in einem ersten Schritt i), durchgeführt unter einem Druck zwischen 100 und 300 Bar, und in einem Schritt ii), durchgeführt unter einem Druck zwischen 10 und 60 Bar,
c) Sprühtrocknung des aus Schritt b) erhaltenen Gemisches, und
d) Wiedergewinnung der aus Schritt c) erhaltenen Zusammensetzung in Pulverform.

8. Verfahren zur Herstellung einer Nährstoffzusammensetzung für Säuglinge oder Kinder in Pulverform gemäß Anspruch 7, **dadurch gekennzeichnet, dass** die wenigstens zwei verschiedenartigen Pektine wenigstens einem niedrig methylierten Pektin, insbesondere einem niedrig methylierten und amidierten Pektin, dessen Methylierungsgrad zwischen 20% und 50% beträgt, und wenigstens einem hoch methylierten Pektin, dessen Methylierungsgrad zwischen 50% und 90% beträgt, entsprechen.

9. Verfahren zur Herstellung einer Nährstoffzusammensetzung für Säuglinge oder Kinder in Pulverform gemäß Anspruch 7 oder 8, das außerdem einen Schritt der Anwendung, auf die aus Schritt a) oder aus Schritt b) erhaltene flüssige Basis, einer thermischen Behandlung bei einer Temperatur zwischen 60°C und 110°C in einem für die Pasteurisierung besagter Basis hinreichenden Zeitraum umfasst und die Wiedergewinnung einer pasteurisierten Zusammensetzung in Pulverform aus Schritt d) erlaubt.

10. Verfahren gemäß einem der Ansprüche 7 bis 9, **dadurch gekennzeichnet, dass** der Trockenmassegehalt der flüssigen Basis wenigstens 35 Gew.-% bezogen auf das Gesamtgewicht der flüssigen Basis beträgt.

11. Verfahren gemäß einem der Ansprüche 7 bis 10, **dadurch gekennzeichnet, dass** die konstitutiven Bestandteile der Zusammensetzung außerdem vorerhitzte und/oder vorgelatinierte Stärke umfassen.

12. Nährstoffzusammensetzung für Säuglinge oder Kinder in Pulverform, **dadurch gekennzeichnet, dass** sie mit einem Verfahren gemäß einem der Ansprüche 7 bis 11 erhalten werden kann.

13. Zusammensetzung gemäß Anspruch 1 oder 12 zur Verwendung in der Prävention oder Behandlung von Regurgitationen und/oder Reflux beim Säugling oder beim Kind.

14. Zusammensetzung zur Verwendung gemäß Anspruch 13 in der Prävention oder Behandlung außerdem von Darmstörungen beim Säugling oder beim Kind.

## Claims

1. A nutritional composition for infant or child, **characterized in that** said composition comprises cold-soluble carob having solubility in an aqueous medium of more than 60% at a temperature comprised between 10°C and 45°C at a concentration comprised between 0.2% and 0.75% by weight and at least two pectins of different natures, including 0.1% to 5% by weight of highly esterified pectins and 1 to 8% by weight of weakly esterified pectins, said percentages being expressed by weight based on the total weight of the composition.

2. The composition according to claim 1, **characterized in that** the pectins are highly methylated pectins, the degree of methylation of which is between 50% and 90%, weakly methylated pectins, in particular amidated and weakly methylated pectins, the degree of methylation of which is between 20% and 50%, or a mixture thereof.

3. The composition according to anyone of the preceding claims, **characterized in that** it further comprises starch.

4. The composition according to anyone of the preceding claims, **characterized in that** it further comprises carbohydrates, said carbohydrates preferably being lactose and/or maltodextrins; lipids; proteins, said proteins being hydrolyzed or non-hydrolyzed; and/or amino acids.

5. The composition according to any one of the preceding claims, **characterized in that** it is as a powder.

6. The composition according to any one of the preceding claims, **characterized in that** the composition is an infant milk, a food supplement for infants, a follow-up milk, a growth milk or a dietetic food.

7. A method for preparing a nutritional composition for infant or child as a powder, said method comprising the steps of:
a) preparing a liquid composition base, for which the dry material content is of at least 20% by weight based on the total weight of said composition, by mixing, with stirring, at a temperature of at least 60°C, the constitutive elements of said composition, said elements comprising at least two pectins of different nature, including 0.1% to 5% by weight of highly esterified pectins and 1 to 8% by weight of weakly esterified pectins and cold-soluble carob having solubility in an aqueous medium of more than 60% at a temperature comprised between 10°C and 45°C, the cold-soluble carob concentration being between 0.2% and 0.75% by weight, said percentages being expressed based on the total weight of the composition,
b) homogenizing the liquid base obtained at the end of step a) by fractionation of the constitutive elements during a first step i) carried out under a pressure between 100 and 300 bars and during a step ii) carried out under a pressure between 10 and 60 bars,
c) drying by atomization of the mixture obtained at the end of step b), and
d) recovering the composition obtained at the end of step c) as a powder.

8. The method for preparing a nutritional composition for infant or child as a powder according to claim 7, **characterized in that** the at least two pectins of different natures correspond to at least one weakly methylated pectin, in particular an amidated and weakly methylated pectin, the degree of methylation of which is between 20% and 50%, and to at least one highly methylated pectin the degree of methylation of which is between 50% and 90%.

9. The method for preparing a nutritional composition for infant or child as a powder according to claim 7 or 8, further comprising a step for applying to the liquid base obtained at the end of step a) or at the end of step b), a heat treatment at a temperature comprised between 60°C and 110°C for a sufficient time in order to pasteurize said base, and allowing recovery of a pasteurized composition as a powder at the end of step d).

10. The method according to any one of claims 7 to 9, **characterized in that** the dry material content of the liquid base is of at least 35% by weight based on the total weight of the liquid base.

11. The method according to any one of claims 7 to 10, **characterized in that** the constitutive elements of the composition further comprise pre-cooked and/or pre-gelatinized starch.

12. A nutritional composition for infant or child as a powder, **characterized in that** it can be obtained by a method according to any one of claims 7 to 11.

13. The composition according to claim 1 or 12, for use in the prevention or treatment of regurgitations and/or reflux of the infant or of the child.

14. The composition for use according to claim 13, in the prevention or treatment in addition of intestinal disorders of the infant or of the child.
